# EUROPEAN PATENT APPLICATION

(11) **EP 4 309 569 A1**
(43) Date of publication of application: **24.01.2024**
(21) Application number: 22794472.5
(22) Date of filing: 29.03.2022
(51) Int. Cl.: A61B 5/00

(54) **ELECTRONIC DEVICE, PATCH AND MEASURING SYSTEM**

(30) Priority: 27.04.2021 CN 202110461872
(71) Applicant: Huawei Technologies Co., Ltd., Longgang Shenzhen, Guangdong 518129 (CN)
(72) Inventor: XU, Gang, Shenzhen, Guangdong 518129 (CN); XIE, Songlin, Shenzhen, Guangdong 518129 (CN); LIU, Xiangyu, Shenzhen, Guangdong 518129 (CN); YAN, Jiabing, Shenzhen, Guangdong 518129 (CN); CHEN, Wenjuan, Shenzhen, Guangdong 518129 (CN)
(74) Representative: Isarpatent
(86) International application number: PCT/CN2022/083850
(87) International publication number: WO 2022/227999

(57) **Abstract**

An electronic device, a patch, and a detection system are provided, to simplify a structure of the patch, and reduce use costs. The detection system is configured to detect a physiological parameter, and includes a patch (10) and an electronic device (20). The patch (10) includes a substrate (14), a first electrode interface (12), an adhesive layer (15), and a first microneedle (13). The first electrode interface (12) is disposed on a first surface of the substrate (14). The adhesive layer (15) is disposed on a second surface of the substrate (14), and the second surface faces away from the first surface. The first microneedle (13) is disposed on the second surface of the substrate. A first electrode (11) is disposed on the first microneedle (13), and the first electrode (11) is coupled to the first electrode interface (12). The electronic device (20) includes an electrochemical sensing circuit (22) and a first external interface (21). The first external interface (21) is disposed on a back surface of the electronic device (20), and the first external interface (21) is coupled to the electrochemical sensing circuit (22). When the electronic device (20) is worn with the back surface of the electronic device (20) facing a human body, the first external interface (21) and the first electrode interface (12) are electrically connected, and an electrical signal is transmitted between the patch (10) and the electronic device (20).

## Description

This application claims priority to Chinese Patent Application No. 202110461872.8, filed with the China National Intellectual Property Administration on April 27, 2021 and entitled "ELECTRONIC DEVICE, PATCH, AND DETECTION SYSTEM", which is incorporated herein by reference in its entirety.

### TECHNICAL FIELD

This application relates to the field of electronic technologies, and in particular, to an electronic device, a patch, and a detection system.

### BACKGROUND

Currently, a quantity of diabetics in the worldwide is increasing rapidly, and diabetes and its complications bring a heavy burden to human health and social development.

Portable or wearable blood glucose detection products emerge, to provide more convenient blood glucose detection manners for the diabetics. Usually, the blood glucose detection product includes a lancet, a blood glucose meter, and a test strip. When using the blood glucose detection product, a user first uses the lancet to break skin at a fingertip to obtain blood of the fingertip, then dips a small amount of blood with the test strip, and finally inserts the test strip into the blood glucose meter. After about 10 seconds, a screen of the blood glucose meter displays a blood glucose level. The blood glucose detection product often fails to continuously detect blood glucose, and a patient needs to puncture a finger for each measurement, causing pain to the patient.

Currently, a blood glucose detection product includes a patch and a reader/writer. The patch includes a needle-shaped glucose sensing electrode, a potentiostat circuit module, a data processing module, and a transmission module. The patch may be worn on a human body for detecting blood glucose. The blood glucose detection product can continuously detect the blood glucose, and the patient does not need to puncture a finger for each measurement. However, the glucose sensing electrode of the patch needs to be placed inside human skin. To avoid infection caused by long-term placement of the glucose sensing electrode into the skin (or failure of normal use of the glucose sensing electrode), the patch usually has a given service life after being applied to the abdomen of the patient, for example, the patch needs to be replaced after three to five days at most. Therefore, as a consumable, the glucose sensing electrode, the potentiostat circuit module, the data processing module, and the transmission module are all integrated on the patch, and as a result, a structure of the patch is complex, and use costs are increased.

### SUMMARY

Embodiments of this application provide an electronic device, a patch, and a detection system, to simplify a structure of the patch, and reduce use costs.

To achieve the foregoing objective, this application uses the following technical solutions.

According to a first aspect, a detection system is provided. The detection system is used to detect physiological parameters such as blood glucose, blood lipids, and blood oxygen. The detection system includes a patch and an electronic device. The patch includes a substrate, a first electrode interface, an adhesive layer, and a first microneedle. The first electrode interface is disposed on a first surface of the substrate. The adhesive layer is disposed on a second surface of the substrate, and the second surface faces away from the first surface. The first microneedle is disposed on the second surface of the substrate. A first electrode is disposed on the first microneedle, and the first electrode is coupled to the first electrode interface. The electronic device includes an electrochemical sensing circuit and a first external interface. The first external interface is disposed on a back surface of the electronic device, and the first external interface is coupled to the electrochemical sensing circuit. When the electronic device is worn with the back surface of the electronic device facing a human body, the first external interface and the first electrode interface are electrically connected, and an electrical signal is transmitted between the patch and the electronic device. In this way, when the patch is applied to the human body, the first microneedle pierces into dermis of the human body, so that the first electrode can be in contact with blood of the dermis. When the electronic device is worn on the human body (for example, the wrist), the electrical connection between the first external interface of the electronic device and the first electrode interface of the patch is formed. The electrical signal may be transmitted between the patch and the electronic device. In this way, when components (such as the glucose, the oxygen, and the blood lipids) in the blood generate electrochemical reactions at the first electrode to form electrical signals, the electrochemical sensing circuit connected to the first external interface of the electronic device can detect the electrical signal formed through the electrochemical reaction. Then, the electrical signal is converted into a detection signal to detect the physiological parameter. In this way, because no circuit module such as the electrochemical sensing circuit, a data processing module, and a transmission module is disposed on the patch, a structure of the patch is simplified, and use costs are reduced.

In a possible implementation, a second electrode is further disposed on the first microneedle, the patch further includes a second electrode interface, and the second electrode interface is disposed on the first surface of the substrate. The second electrode is coupled to the second electrode interface. The electronic device further includes a second external interface, the second external interface is disposed on the back surface of the electronic device, and the second external interface is coupled to the electrochemical sensing circuit. When the electronic device is worn with the back surface of the electronic device facing the human body, the second external interface and the second electrode interface are electrically connected, and an electrical signal is transmitted between the patch and the electronic device. Detecting different physiological parameters may need to be implemented by using one or more electrodes. In this case, the one or more electrodes may be disposed on the first microneedle. For example, for blood glucose detection, two different electrodes, for example, the first electrode and the second electrode, may be disposed on the first microneedle, and the first electrode and the second electrode may be a work electrode and a reference electrode respectively. Certainly, for the blood glucose detection, three electrodes may alternatively be disposed on the first microneedle, for example, a counter electrode may be further disposed.

In a possible implementation, the patch further includes a second microneedle and a second electrode interface. The second microneedle is disposed on the second surface of the substrate. The second electrode interface is disposed on the first surface of the substrate. A second electrode is disposed on the second microneedle, and the second electrode is coupled to the second electrode interface. The electronic device further includes a second external interface, the second external interface is disposed on the back surface of the electronic device, and the second external interface is coupled to the electrochemical sensing circuit. When the electronic device is worn with the back surface of the electronic device facing the human body, the second external interface and the second electrode interface are electrically connected, and an electrical signal is transmitted between the patch and the electronic device. Detecting different physiological parameters may need to be implemented by using one or more electrodes. In this case, a plurality of microneedles may be disposed. For example, the second microneedle is disposed on the second surface of the substrate, and the second electrode is disposed on the second microneedle. For example, for blood glucose detection, the first electrode may be disposed on the first microneedle, the second electrode may be disposed on the second microneedle, and the first electrode is different from the second electrode. For example, the first electrode and the second electrode may be a work electrode and a reference electrode respectively. Certainly, for the blood glucose detection, the patch may further include a counter electrode. The counter electrode may be disposed on the first electrode or the second electrode, or the counter electrode may be separately disposed on a third microneedle on the second surface of the substrate.

In a possible implementation, the first electrode interface and the second electrode interface have magnetism, or the first electrode interface and the second electrode interface are magnetic materials. The first external interface and the second external interface have magnetism, or the first external interface and the second external interface are magnetic materials. For example, the first external interface and the first electrode interface (the second external interface and the second electrode interface) may use magnetic materials such as neodymium iron boron, samarium cobalt, aluminum nickel cobalt, and iron chromium cobalt, and the magnetic materials may be magnetized by using a magnetic field, so that the magnetic materials have magnetism. The first external interface and the first electrode interface (the second external interface and the second electrode interface of the patch) may be magnetically engaged to ensure reliability of the electrical connection between the first external interface of the electronic device and the first electrode interface of the patch (between the second external interface and the second electrode interface of the patch). Specifically, both the first external interface and the first electrode interface (the second external interface and the second electrode interface) may have the magnetism (for example, opposite magnetism). Alternatively, one has the magnetism, and the other is the magnetic material. In addition, when both the first external interface and the first electrode interface (the second external interface and the second electrode interface) use the magnetic materials, one or more layers of materials with high conductivity such as nickel and copper may be manufactured, through electroplating or metal deposition, on surfaces on which the first external interface and the first electrode interface (the second external interface and the second electrode interface) are in contact with each other, to increase conductivity of the first external interface and the first electrode interface (the second external interface and the second electrode interface). In this way, because the first external interface and the first electrode interface (the second external interface and the second electrode interface) are engaged in a weak magnetic mode, the reliability of the electrical connection between the first external interface and the first electrode interface can be ensured. When an external force disturbs the electronic device, the first external interface and the first electrode interface (the second external interface and the second electrode interface) may be detached to avoid a case in which the disturbance of the electronic device causes the patch to scratch human skin.

In a possible implementation, the patch further includes a water retaining strip. The water retaining strip is disposed on the first surface of the substrate, and the water retaining strip surrounds the first electrode interface. In this way, when the water retaining strip and the electronic device are attached, closed space may be formed between the back surface of the electronic device and the substrate to avoid impact of conductive liquid such as water or sweat stains on the first electrode interface. For example, when the second electrode interface is disposed on the patch, the conductive liquid may short-circuit the first electrode interface and the second electrode interface, thereby affecting reliability of physiological parameter detection.

In a possible implementation, the patch further includes an application mark. The application mark is disposed on the first surface of the substrate, and the application mark indicates an application direction of the patch. For example, when the patch is applied to the skin, the application mark indicates application in a direction between the ring finger and the middle finger. In this way, it is ensured that the first external interface of the electronic device and the first electrode interface of the patch (or the second external interface and the second electrode interface of the patch) are connected to form a correct connection mode.

In a possible implementation, the first electrode interface and the second electrode interface are concentrically disposed ring-shaped electrode interfaces. The first external interface and the second external interface are concentrically disposed ring-shaped external interfaces. It may be understood that, when the first electrode interface and the second electrode interface of the patch are the concentrically disposed ring-shaped electrode interfaces, and the corresponding first external interface and the corresponding second external interface of the electronic device are the concentrically disposed ring-shaped external interfaces, on an axis perpendicular to a center of a ring, rotating the electronic device does not affect the normal connection between the first external interface and the first electrode interface (or between the second external interface and the second electrode interface).

In a possible implementation, a shape of the first electrode includes a columnar shape, a planar shape, or a wire-wound shape. Certainly, when there is another electrode on the patch, the foregoing shape may also be used.

In a possible implementation, the first electrode includes one or more of the following materials: lamp black carbon, glassy carbon, graphite, silver, silver chloride, platinum, palladium, a platinum-iridium alloy, titanium, gold, and iridium. Certainly, when there is another electrode on the patch, the foregoing material may also be used. In particular, a reference electrode RE may use the silver or the silver chloride. Since a silver or silver chloride electrode has little solubility, and extremely high stability and reversibility in an aqueous solution system, and a surface of the electrode is well protected even hydrogen exists, when the silver or silver chloride electrode is used as the reference electrode, self-noise of the electrode can be minimized.

In a possible implementation, to ensure fast implantation, less pain, a small wound surface, and that a length of the microneedle is sufficient for subcutaneous placement, a length of the first microneedle is from 1 to 5 mm, and a diameter of the first microneedle is from 7 to 400 µm. Certainly, when there is another microneedle (for example, the second microneedle) on the patch, the foregoing size of the first microneedle may also be used.

In a possible implementation, a material of the first microneedle includes stainless steel, platinum, or a polymer material. Certainly, when there is another microneedle (for example, the second microneedle) on the patch, the foregoing material of the first microneedle may also be used.

In a possible implementation, the patch further includes a first magnetic interface. The first magnetic interface is disposed on the first surface of the substrate, and the first magnetic interface has magnetism, or the first magnetic interface is a magnetic material. The electronic device further includes a second magnetic interface disposed on the back surface of the electronic device. The second magnetic interface has magnetism, or the second magnetic interface is a magnetic material. When the electronic device is worn with the back surface of the electronic device facing the human body, the first magnetic interface and the second magnetic interface are engaged. For example, the first magnetic interface may be disposed on the first surface of the substrate of the patch. The first magnetic interface has the magnetism, or the first magnetic interface is the magnetic material. The second magnetic interface is disposed on the electronic device. The second magnetic interface has the magnetism, or the second magnetic interface is the magnetic material. One surface of the second magnetic interface exposes the electronic device. Specifically, both the first magnetic interface and the second magnetic interface may have the magnetism (for example, opposite magnetism). Alternatively, one has the magnetism, and the other is the magnetic material. Positions of first magnetic interfaces one-to-one correspond to positions of second magnetic interfaces. After the electronic device and the patch are attached through the first magnetic interface and the second magnetic interface, the first external interface and the first electrode interface (the second external interface and the second electrode interface) are electrically connected.

According to a second aspect, a patch is provided, configured to detect a physiological parameter. The patch includes: a substrate; a first electrode interface, where the first electrode interface is disposed on a first surface of the substrate, and the first electrode interface is configured to: after being electrically connected to an electronic device, transmit an electrical signal between the patch and the electronic device; an adhesive layer, where the adhesive layer is disposed on a second surface of the substrate, and the second surface faces away from the first surface; and a first microneedle, where the first microneedle is disposed on the second surface of the substrate. A first electrode is disposed on the first microneedle, and the first electrode is coupled to the first electrode interface.

In a possible implementation, a second electrode is further disposed on the first microneedle, and the patch further includes a second electrode interface. The second electrode interface is disposed on the first surface of the substrate. The second electrode is coupled to the second electrode interface.

In a possible implementation, the patch further includes: a second microneedle, where the second microneedle is disposed on the second surface of the substrate; and a second electrode interface, where the second electrode interface is disposed on the first surface of the substrate. A second electrode is disposed on the second microneedle, and the second electrode is coupled to the second electrode interface.

In a possible implementation, the first electrode includes any one of a work electrode and a reference electrode.

In a possible implementation, the second electrode includes any one of the work electrode and the reference electrode, and the second electrode is different from the second electrode.

In a possible implementation, the first electrode interface and the second electrode interface have magnetism, or the first electrode interface and the second electrode interface are magnetic materials.

In a possible implementation, the patch further includes a water retaining strip. The water retaining strip is disposed on the first surface of the substrate, and the water retaining strip surrounds the first electrode interface.

In a possible implementation, the patch further includes an application mark. The application mark is disposed on the first surface of the substrate, and the application mark indicates an application direction of the patch.

In a possible implementation, the first electrode interface and the second electrode interface are concentrically disposed ring-shaped electrode interfaces.

In a possible implementation, a shape of the first electrode includes a columnar shape, a planar shape, or a wire-wound shape.

In a possible implementation, the first electrode includes one or more of the following materials: lamp black carbon, glassy carbon, graphite, silver, silver chloride, platinum, palladium, a platinum-iridium alloy, titanium, gold, and iridium.

In a possible implementation, a length of the first microneedle is from 1 to 5 mm, and a diameter of the first microneedle is from 7 to 400 µm.

In a possible implementation, a material of the first microneedle includes stainless steel, platinum, or a polymer material.

In a possible implementation, the patch further includes a first magnetic interface. The first magnetic interface is disposed on the first surface of the substrate, and the first magnetic interface has magnetism, or the first magnetic interface is a magnetic material.

According to a third aspect, an electronic device is provided, configured to detect a physiological parameter. The electronic device includes: an electrochemical sensing circuit; and a first external interface. The first external interface is disposed on a back surface of the electronic device, and the first external interface is coupled to the electrochemical sensing circuit. When the electronic device is worn with the back surface of the electronic device facing a human body, the first external interface and a first electrode interface of a patch are electrically connected, and an electrical signal is transmitted between the patch and the electronic device.

In a possible implementation, the first external interface has magnetism, or the first external interface is a magnetic material.

In a possible implementation, the electronic device further includes a second external interface, the second external interface is disposed on the back surface of the electronic device, and the second external interface is coupled to the electrochemical sensing circuit. When the electronic device is worn with the back surface of the electronic device facing the human body, the second external interface and a second electrode interface of the patch are electrically connected, and an electrical signal is transmitted between the patch and the electronic device.

In a possible implementation, the electronic device further includes a second magnetic interface disposed on the back surface of the electronic device. The second magnetic interface has magnetism, or the second magnetic interface is a magnetic material.

In a possible implementation, the first external interface and the second external interface are concentrically disposed ring-shaped external interfaces.

According to a fourth aspect, a patch in-position detection method is provided, applied to the detection system according to the first aspect. The method includes: determining a connection status of a patch based on a detection signal output by an electrochemical sensor; and when it is determined that a strength of the detection signal is less than or equal to a signal threshold, generating first prompt information, where the first prompt information prompts a user to adjust a wearing position of an electronic device. The detection signal output by an electrochemical sensing circuit may reflect a connection status between a first external interface of the electronic device and a first electrode interface of the patch. For example, if the strength of the detection signal is zero, it indicates that the first external interface of the electronic device and the first electrode interface of the patch are completely disconnected. Alternatively, if the detection signal output is greater than 0 but less than the signal threshold, it indicates that the first external interface of the electronic device is in poor contact with the first electrode interface of the patch, and a high impedance contact problem exists. In this case, a wearing posture of the electronic device needs to be adjusted, so that the first external interface of the electronic device and the first electrode interface of the patch are electrically reconnected.

In a possible implementation, the method further includes: when it is determined that the strength of the detection signal is greater than the signal threshold, generating second prompt information, where the second prompt information prompts the user to start physiological parameter detection.

In a possible implementation, the first prompt information includes vibration, a sound, or display information.

According to a fifth aspect, an electronic device is provided. The electronic device includes a memory and one or more processors. The memory is coupled to the processor, the memory is configured to store computer program code, and the computer program code includes computer instructions. When the processor executes the computer instructions, the electronic device is enabled to perform the method according to any implementation of the fourth aspect.

According to a sixth aspect, a computer-readable storage medium is provided, including computer instructions. When the computer instructions are run on an electronic device, the electronic device is enabled to perform the method according to any implementation of the third aspect.

Technical effects that can be implemented in the second aspect to the sixth aspect are similar to those in the first aspect. Details are not described again.

### BRIEF DESCRIPTION OF DRAWINGS

FIG. 1 is a schematic diagram of a structure of a blood glucose detection product according to the conventional technology;
FIG. 2 is a schematic diagram of a scenario of a detection system according to an embodiment of this application;
FIG. 3 is a schematic diagram of a scenario of a detection system according to another embodiment of this application;
FIG. 4 is a schematic diagram of a structure of an electronic device according to an embodiment of this application;
FIG. 5 is a schematic diagram of a structure of a patch according to an embodiment of this application;
FIG. 6 is a schematic diagram of a structure of an electronic device according to another embodiment of this application;
FIG. 7 is a schematic diagram of a structure of an electrochemical sensing circuit according to an embodiment of this application;
FIG. 8 is a schematic diagram of a structure of a patch according to another embodiment of this application;
FIG. 9 is a schematic diagram of a structure of a patch according to still another embodiment of this application;
FIG. 10 is a schematic diagram of a structure of a patch according to yet another embodiment of this application;
FIG. 11 is a schematic diagram of a structure of a patch according to another embodiment of this application;
FIG. 12 is a schematic diagram of a structure of a microneedle according to an embodiment of this application;
FIG. 13 is a schematic diagram of a structure of a microneedle according to another embodiment of this application;
FIG. 14 is a schematic diagram of a structure of a microneedle according to still another embodiment of this application;
FIG. 15 is a schematic diagram of a structure of a microneedle according to yet another embodiment of this application;
FIG. 16 is a schematic diagram of a structure of a microneedle according to another embodiment of this application;
FIG. 17 is a schematic diagram of a structure of a microneedle according to still another embodiment of this application;
FIG. 18 is a schematic diagram of a structure of a patch according to still another embodiment of this application;
FIG. 19 is a schematic diagram of a structure of a patch according to yet another embodiment of this application;
FIG. 20 is a schematic diagram of a structure of a patch according to another embodiment of this application;
FIG. 21 is a schematic diagram of a structure of a patch according to still another embodiment of this application;
FIG. 22 is a schematic diagram of a structure of a patch according to yet another embodiment of this application;
FIG. 23 is a schematic diagram of a structure of a patch according to another embodiment of this application;
FIG. 24 is a schematic diagram of a structure of a patch according to still another embodiment of this application;
FIG. 25 is a schematic diagram of a structure of a microneedle according to yet another embodiment of this application;
FIG. 26 is a schematic diagram of a structure of an electronic device according to still another embodiment of this application;
FIG. 27 is a schematic diagram of a structure of an electronic device according to yet another embodiment of this application;
FIG. 28 is a schematic diagram of a structure of a patch according to yet another embodiment of this application;
FIG. 29 is a schematic diagram of a structure of a patch according to another embodiment of this application;
FIG. 30 is a schematic diagram of a structure of a patch according to still another embodiment of this application;
FIG. 31 is a schematic diagram of a structure of a patch according to yet another embodiment of this application;
FIG. 32 is a schematic diagram of a structure of an electronic device according to another embodiment of this application;
FIG. 33 is a schematic diagram of a structure of a patch according to another embodiment of this application;
FIG. 34 is a schematic diagram of a structure of a patch according to still another embodiment of this application;
FIG. 35 is a schematic diagram of a structure of a coating of a work electrode according to an embodiment of this application;
FIG. 36 is a schematic diagram of a working principle of a coating of a work electrode according to an embodiment of this application;
FIG. 37 is a schematic diagram of a structure of a coating of a work electrode according to another embodiment of this application;
FIG. 38 is a schematic diagram of a working principle of a coating of a work electrode according to another embodiment of this application;
FIG. 39 is a schematic flowchart of a patch in-position detection method according to an embodiment of this application;
FIG. 40 is a schematic diagram 1 of a display interface of a smartwatch according to an embodiment of this application;
FIG. 41 is a schematic diagram 2 of a display interface of a smartwatch according to an embodiment of this application;
FIG. 42 is a schematic diagram 3 of a display interface of a smartwatch according to an embodiment of this application;
FIG. 43 is a schematic diagram 4 of a display interface of a smartwatch according to an embodiment of this application;
FIG. 44 is a schematic diagram 5 of a display interface of a smartwatch according to an embodiment of this application;
FIG. 45 is a schematic diagram 6 of a display interface of a smartwatch according to an embodiment of this application;
FIG. 46 is a schematic diagram of a structure of an electronic device according to still another embodiment of this application; and
FIG. 47 is a schematic diagram of a structure of a chip system according to an embodiment of this application.

### DESCRIPTION OF EMBODIMENTS

The following describes the technical solutions in embodiments of this application with reference to the accompanying drawings in embodiments of this application. It is clear that the described embodiments are merely a part rather than all of embodiments of this application.

Unless defined otherwise, all technical and scientific terms used herein have the same meaning as commonly understood by a person of ordinary skill in the art to which this disclosure belongs. In this application, "at least one" means one or more, and "a plurality of" means two or more. "And/or" describes an association relationship between associated objects, and indicates that three relationships may exist. For example, A and/or B may indicate the following cases: Only A exists, both A and B exist, and only B exists, where A and B may be singular or plural. The character "/" generally indicates an "or" relationship between the associated objects. "At least one of the following items (pieces)" or a similar expression thereof refers to any combination of these items, including a single item (piece) or any combination of a plurality of items (pieces). For example, at least one of a, b, or c may represent: a, b, c, a and b, a and c, b and c, or a, b, and c, where a, b, and c may be singular or plural. In addition, in embodiments of this application, the terms such as "first" and "second" are not intended to limit a quantity or an execution sequence.

In addition, in this application, position terms such as "top" and "bottom" are defined relative to positions of components in the accompanying drawings. It should be understood that these position terms are relative concepts used for relative description and clarification, and may correspondingly change based on changes in the positions of the components in the accompanying drawings.

It should be noted that, in this application, terms such as "an example" or "for example" indicate an example, an instance, or an illustration. Any embodiment or design scheme described as an "example" or "for example" in this application should not be explained as being more preferred or having more advantages than another embodiment or design scheme. Exactly, use of the word such as "example" or "for example" is intended to present a related concept in a specific manner.

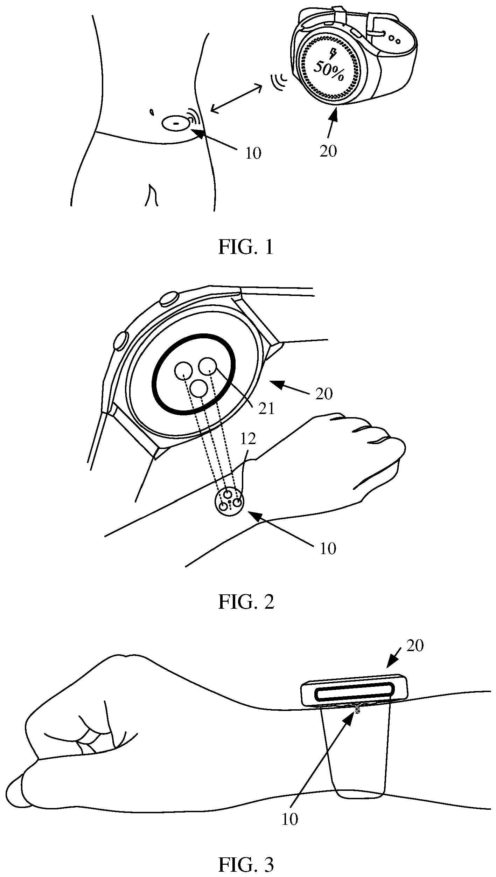

A blood glucose detection product includes a patch and a reader/writer. The patch includes a needle-shaped glucose sensing electrode, a potentiostat circuit module, a data processing module, and a transmission module. The patch may be worn on a human body. As shown in FIG. 1, the patch 10 is directly applied on the abdomen of a patient (certainly, the patch 10 may alternatively be applied to another part, such as the upper arm, of the human body). In this way, the glucose sensing electrode can pierce into skin of the patient (for example, the glucose sensing electrode pierces epidermis of the skin into dermis, and is in contact with blood, so that glucose in the blood generates an electrochemical reaction at the glucose sensing electrode). The potentiostat circuit module can detect an electrical signal generated through the electrochemical reaction at the glucose sensing electrode, and convert the electrical signal into a digital signal through processing by the data processing module. Finally, the transmission module converts the digital signal into a radio frequency signal, and sends the radio frequency signal to the reader/writer 20 (which may be a smart wearable device, such as a watch or a dedicated blood glucose meter). In this way, the reader/writer 20 can obtain and display blood glucose data of the patch. The glucose sensing electrode of the patch 10 needs to be placed inside human skin. To avoid infection caused by long-term placement of the glucose sensing electrode into the skin (or failure of normal use of the glucose sensing electrode), the patch 10 usually has a given service life after being applied to the abdomen of the patient, for example, the patch needs to be replaced after three to five days at most. Therefore, as a consumable, the glucose sensing electrode, the potentiostat circuit module, the data processing module, and the transmission module are all integrated on the patch 10, and as a result, a structure of the patch is complex, and use costs are increased.

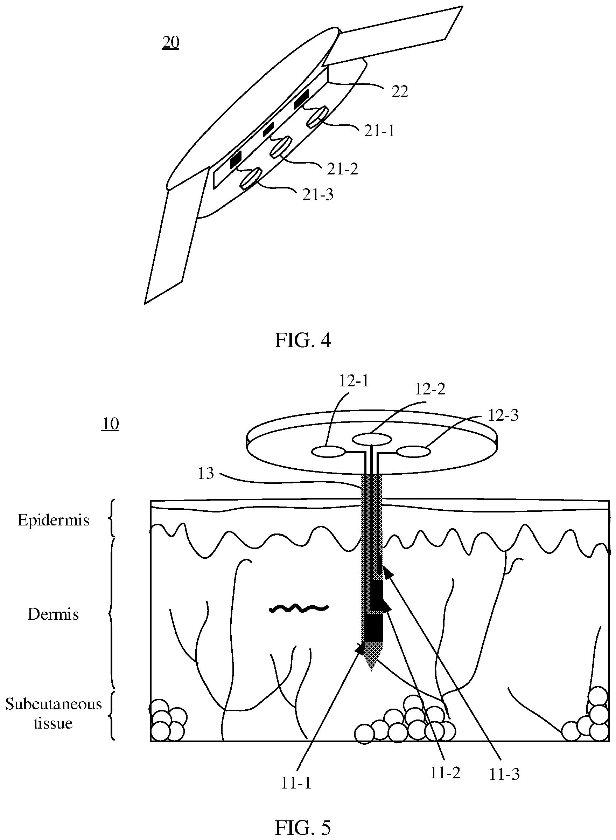

To resolve the foregoing problem, with reference to application scenarios shown in FIG. 2, FIG. 3, FIG. 4, and FIG. 5, principles of the solutions provided in embodiments of this application are briefly described as follows: Embodiments of this application provide an electronic device, a patch, and a detection system that is mainly used to detect any physiological parameter including blood glucose, blood lipids, blood oxygen, and the like. In this embodiment of this application, circuit modules such as an electrochemical sensing circuit, the data processing module, and the transmission module do not need to be disposed on the patch 10. Only a microneedle 13 configured to be placed inside the human skin and electrodes 11 (three electrodes 11-1, 11-2, and 11-3 are shown in FIG. 5) on the microneedle 13 are disposed on the patch 10. The circuit modules such as the electrochemical sensing circuit 22 (as shown in FIG. 4) and the data processing module are disposed on the electronic device 20 (for example, may be a wearable device such as a smartwatch). At least two external interfaces 21 (FIG. 4 shows three external interfaces 21-1, 21-2, and 21-3) coupled to the electrochemical sensing circuit 22 are disposed on a back surface (as shown in FIG. 3, a surface facing the human body during normal wear) of the electronic device 20. In addition, electrode interfaces 12 (three electrode interfaces 12-1, 12-2, and 12-3 are shown in FIG. 5) coupled to the electrode 11 are disposed on the patch 10. In this way, when the patch 10 is applied to the human body (as shown in FIG. 5), the microneedle 15 pierces into the dermis of the human body, so that the electrodes 11 can be in contact with blood of the dermis. When the electronic device 20 is worn on the human body (for example, the wrist, as shown in FIG. 3), electrical connections between the external interfaces 21 of the electronic device 20 and the electrode interfaces 12 of the patch 10 are formed (for example, electrical connection relationships are shown by dashed lines in FIG. 2). An electrical signal may be transmitted between the patch 10 and the electronic device 20. In this way, components (such as the glucose, the oxygen, and the blood lipids) in the blood generate electrochemical reactions at the electrodes 11 to form electrical signals. Specifically, for blood glucose detection, the glucose in the blood may generate the electrochemical reaction at a coating coated on a work electrode of the electrodes 11, to form the electrical signal. In this way, the electrochemical sensing circuit 22 can detect the electrical signal formed through the electrochemical reaction, and the electronic device 20 further converts the electrical signal into a detection signal to detect the physiological parameter. In this way, because no circuit module such as the electrochemical sensing circuit, the data processing module, and the transmission module is disposed on the patch 10, the structure of the patch 10 is simplified, and use costs are reduced. In addition, in some solutions, at least one of the external interfaces 21 and the electrode interfaces 12 may be disposed as magnetic interfaces. For example, a magnetic material may be used to magnetically engage the external interfaces 21 with the electrode interfaces 12, to ensure reliability of the electrical connections between the external interfaces 21 and the electrode interfaces 12. In addition, when the electronic device 20 is disturbed by an external force, the external interfaces 21 and the electrode interfaces 12 may be detached to avoid a case in which the disturbance of the electronic device 20 causes the patch 10 to scratch the human skin. Certainly, the magnetic interfaces may also be separately disposed on the electronic device 20 and the patch 10 to engage the electronic device 20 with the patch 10. Principles and main technical solutions of products provided in embodiments of this application are briefly described above, and are described in detail below with reference to specific examples.

For example, the electronic device in embodiments of this application may be a wearable device such as a smartwatch, a smart wristband, or a smart belt. A specific form of the electronic device is not specially limited in embodiments of this application.

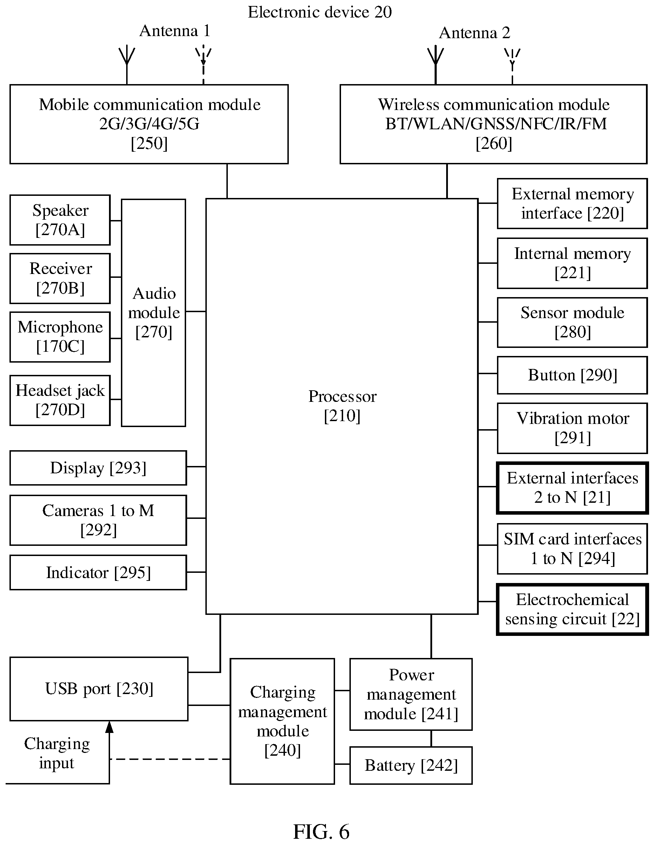

The following describes the implementations of embodiments of this application in detail with reference to accompanying drawings. FIG. 6 is a schematic diagram of a structure of the electronic device 20 according to an embodiment of this application. As shown in FIG. 2, the electronic device 20 may include a processor 210, an external memory interface 220, an internal memory 221, a universal serial bus (universal serial bus, USB) port 230, a charging management module 240, a power management module 241, a battery 242, an antenna 1, an antenna 2, a mobile communication module 250, a wireless communication module 260, an audio module 270, a speaker 270A, a receiver 270B, a microphone 270C, a headset jack 270D, a sensor module 280, a button 290, a vibration motor 291, the external interfaces 21, a camera 292, a display 293, a subscriber identification module (subscriber identification module, SIM) card interface 294, the electrochemical sensing circuit 22, an indicator 295, and the like.

It may be understood that an example structure in this embodiment does not constitute a specific limitation on the electronic device 20. In some other embodiments, the electronic device 20 may include more or fewer components than those shown in the figure, or some components may be combined, or some components may be split, or there may be a different component layout. The components shown in the figure may be implemented by using hardware, software, or a combination of software and hardware. It may be understood that an interface connection relationship between modules illustrated in this embodiment is merely an example for description, and does not constitute a limitation on the structure of the electronic device 20. In some other embodiments, the electronic device 20 may alternatively use an interface connection mode different from that in the foregoing embodiment, or a combination of a plurality of interface connection modes.

The processor 210 may include one or more processing units. For example, the processor 210 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and/or a neural-network processing unit (neural-network processing unit, NPU). Different processing units may be independent components, or may be integrated into one or more processors. The processor may be a nerve center and a command center of the electronic device 20. The processor may generate an operation control signal based on an instruction operation code and a time sequence signal to complete control of instruction fetching and instruction execution.

The memory may be further disposed in the processor 210, and is configured to store instructions and data. In some embodiments, the memory in the processor 210 is a cache. The memory may store instructions or data just used or cyclically used by the processor 210. If the processor 210 needs to use the instructions or the data for another time, the processor 210 may directly invoke the instructions or the data from the memory. This avoids repeated access and reduces waiting time of the processor 210, thereby improving system efficiency.

In some embodiments, the processor 210 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The external memory interface 220 may be configured to connect to an external storage card, for example, a micro SD card, to extend a storage capability of the electronic device 20. The external storage card communicates with the processor 210 through the external memory interface, to implement a data storage function. For example, files such as music and video are stored in the external storage card.

The internal memory 221 may be configured to store computer-executable program code, where the executable program code includes instructions. The processor 210 executes various function applications and data processing of the electronic device 20 by running the instructions stored in the internal memory 221. For example, in this embodiment of this application, the processor 210 may execute the instructions stored in the internal memory 221, and the internal memory 221 may include a program storage area and a data storage area.

The program storage area may store an operating system, an application required by at least one function (for example, a voice playing function or an image playing function), and the like. The data storage area may store data (such as audio data and a phone book) created during use of the electronic device 20, and the like. In addition, the internal memory 221 may include a high-speed random access memory, or may include a non-volatile memory, for example, at least one magnetic disk storage device, a flash memory, or a universal flash storage (universal flash storage, UFS).

The charging management module 240 is configured to receive a charging input from a charger. The charger may be a wireless charger or a wired charger. In some embodiments of wired charging, the charging management module 240 may receive a charging input of a wired charger through the USB port 230. In some embodiments of wireless charging, the charging management module 240 may receive a wireless charging input through a wireless charging coil of the electronic device 20. The charging management module 240 may further supply power to the electronic device 20 by using the power management module 241 when the battery 241 is charged.

The power management module 241 is configured to connect to the battery 241, the charging management module 240, and the processor 210. The power management module 241 receives an input of the battery 241 and/or the charging management module 240, and supplies power to the processor 210, the internal memory 221, an external memory, the display 293, the camera 292, the wireless communication module 260, and the like. The power management module 241 may be further configured to monitor parameters such as a battery capacity, a quantity of battery cycles, and a battery health status (electric leakage and impedance). In some other embodiments, the power management module 241 may alternatively be disposed in the processor 210. In some other embodiments, the power management module 241 and the charging management module 240 may alternatively be disposed in a same device.

A wireless communication function of the electronic device 20 may be implemented through the antenna 1, the antenna 2, the mobile communication module 250, the wireless communication module 260, the modem processor, the baseband processor, and the like.

The antenna 1 and the antenna 2 are configured to transmit and receive electromagnetic wave signals. Each antenna in the electronic device 20 may be configured to cover one or more communication bands. Different antennas may be further multiplexed, to improve antenna utilization. For example, the antenna 1 may be multiplexed as a diversity antenna in a wireless local area network. In some other embodiments, an antenna may be used in combination with a tuning switch.

The mobile communication module 250 may provide a wireless communication solution that includes 2G/3G/4G/5G or the like and that is applied to the electronic device 20. The mobile communication module 250 may include at least one filter, a switch, a power amplifier, a low noise amplifier (low noise amplifier, LNA), and the like.

The wireless communication module 260 may provide a solution that is applied to the electronic device 20 and that is for wireless communication such as a wireless local area network (wireless local area network, WLAN), Bluetooth (Bluetooth, BT), a global navigation satellite system (global navigation satellite system, GNSS), frequency modulation (frequency modulation, FM), near field communication (near field communication, NFC), or an infrared (infrared, IR) technology. For example, the WLAN may be a wireless fidelity (wireless fidelity, Wi-Fi) network.

The wireless communication module 260 may be one or more components integrating at least one communication processing module. The wireless communication module 260 receives an electromagnetic wave through the antenna 2, performs frequency modulation and filtering processing on an electromagnetic wave signal, and sends a processed signal to the processor 210. The wireless communication module 260 may further receive a to-be-sent signal from the processor 210, perform frequency modulation and amplification on the signal, and convert the signal into an electromagnetic wave for radiation through the antenna 2.

In some embodiments, in the electronic device 20, the antenna 2 and the mobile communication module 250 are coupled, and the antenna 2 and the wireless communication module 260 are coupled, so that the electronic device 20 can communicate with a network and another device by using a wireless communication technology.

The electronic device 20 implements a display function by using the GPU, the display 293, the application processor, and the like. The GPU is a microprocessor for image processing, and is connected to the display 293 and the application processor. The GPU is configured to: perform mathematical and geometric calculation, and render an image. The processor 210 may include one or more GPUs that execute program instructions to generate or change display information. The display 293 may be a touchscreen, and the display 293 is configured to display an image, a video, and the like. The display 292 includes a display panel.

The electronic device 20 may implement a photographing function by using the ISP, the camera 292, the video codec, the GPU, the display 293, the application processor, and the like. The ISP is configured to process data fed back by the camera 292. In some embodiments, the ISP may be disposed in the camera 292. The camera 292 is configured to capture a static image or video. In some embodiments, the electronic device 20 may include one or M cameras 292, where M is a positive integer greater than 1.

The electronic device 20 may implement audio functions, for example, a music playing function and a recording function, by using the audio module 270, the speaker 270A, the receiver 270B, the microphone 270C, the headset jack 270D, the application processor, and the like.

The audio module 270 is configured to convert digital audio information into an analog audio signal for output, and is also configured to convert an analog audio input into a digital audio signal. The headset jack 270D is configured to connect to a wired headset. The headset jack 270D may be the USB port 230, or may be a 3.5 mm open mobile electronic terminal platform (open mobile terminal platform, OMTP) standard interface or a cellular telecommunications industry association of the USA (cellular telecommunications industry association of the USA, CTIA) standard interface.

The sensor module 280 may include a gyro sensor, a barometric pressure sensor, a magnetic sensor, an acceleration sensor, a distance sensor, an optical proximity sensor, a fingerprint sensor, a temperature sensor, a touch sensor, an ambient light sensor, a bone conduction sensor, and the like.

The button 290 includes a power button, a volume button, and the like. The button 290 may be a mechanical button, or may be a touch button. The electronic device 20 may receive a button input, and generate a button signal input related to a user setting and function control of the electronic device 20.

The vibration motor 291, which may also be referred to as a motor, may be used for a vibration prompt. The vibration motor 291 may be used for an incoming call vibration prompt. For example, touch operations performed on different applications (for example, photographing and audio playing) may correspondingly generate different vibration feedback effects. The vibration motor 291 may also correspond to different vibration feedback effects for touch operations performed on different areas of the display 293. Different application scenarios (for example, a time reminder, information receiving, an alarm clock, a game) may also correspond to different vibration feedback effects. Touch vibration feedback effects may be further customized.

The vibration motor 291, the display 293, and the speaker 270A in this embodiment of this application may be configured to display, to a user, first prompt information that prompts the user to adjust a wearing position of the electronic device 20, or second prompt information that prompts the user to start physiological parameter detection.

The external interfaces 21 are configured to connect to an external device. For example, according to the patch provided in this embodiment of this application, a form of the external interface may be an electrode plate, an electrode block, a conductive block bump, or the like. The electronic device 20 may support two or N external interfaces, where N is a positive integer greater than 2.

The SIM card interface 294 is configured to connect to a SIM card. The SIM card may be inserted into the SIM card interface 294 or detached from the SIM card interface 294, to implement contact with or separation from the electronic device 20. The electronic device 20 may support one or N SIM card interfaces, where N is a positive integer greater than 1. The SIM card interface 294 may support a nano-SIM card, a micro-SIM card, a SIM card, and the like. It may be understood that, when the electronic device 20 is a wearable device that does not have a mobile communication function such as 2G/3G/4G/5G, the SIM card interface 294 and the mobile communication module 250 may not be disposed on the wearable device. In other words, the SIM card interface 294 and the mobile communication module 250 may be optional components.

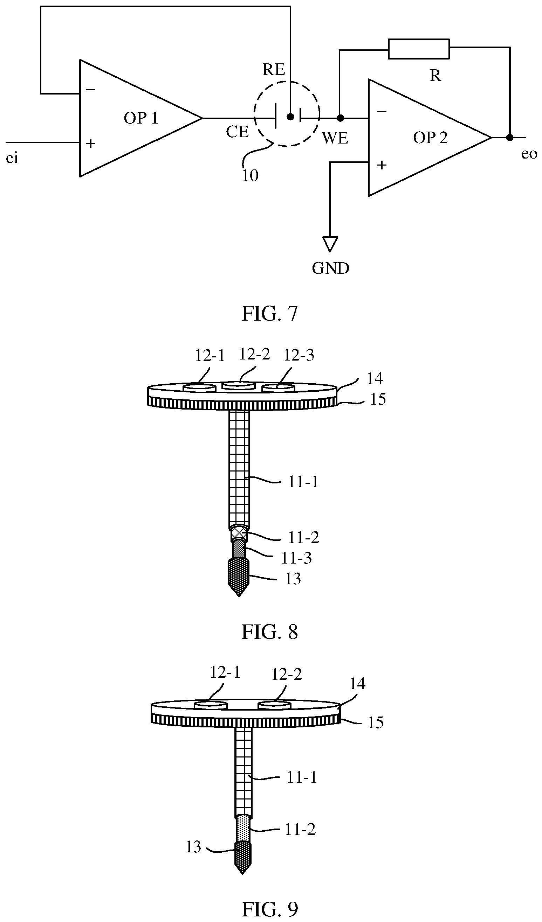

The electrochemical sensing circuit 22 may include an electrical signal detection circuit based on a plurality of electrochemical methods such as amperometry, chronoamperometry, cyclic voltammetry, differential pulse voltammetry, and an alternating current impedance method. With reference to FIG. 7, a schematic diagram of a structure of the electrochemical sensing circuit 22 is provided. The electrochemical sensing circuit 22 includes an operational amplifier OP 1 and an operational amplifier OP 2. A negative end (-) of the operational amplifier OP 1 and a reference electrode (reference electrode, RE) of the patch 10 are connected. An output end of the operational amplifier OP 1 and a counter electrode (counter electrode, CE) of the patch 10 are connected. A negative end (-) of the operational amplifier OP 2 and an output end of the operational amplifier OP 2 are connected by using a resistor R. The negative end (-) of the operational amplifier OP 2 is further connected to a work electrode (work electrode, WE) of the patch 10. A positive end (+) of the operational amplifier OP 2 and a ground end GND are connected. A function of the operational amplifier OP 1 is providing a feedback. A voltage of the RE is equal to a voltage ei input at the positive end (+) of the operational amplifier OP 1 by using "imaginary short" of the operational amplifier OP 1, and there is no current passing through the negative end (-) that is connected to the RE and that is of the OP 1 due to "imaginary short", so that the current passes through only the two electrodes WE and CE. A function of the OP 2 is implementing mutual impedance amplification, and converting the current passing through the WE and the CE into a voltage eo by using R. Certainly, the patch 10 is described by using three electrodes as an example. When the patch 10 includes two electrodes: the WE and the CE, the RE may be directly shortcircuited with the CE. Certainly, the foregoing electrochemical sensing circuit 22 for blood glucose detection is used as an example. When the electrochemical sensing circuit 22 is configured to detect another physiological parameter, the electrochemical sensing circuit 22 may alternatively have another structure.

The indicator 295 may be an indicator light, and may be configured to indicate a charging status and a power change, or may be configured to indicate a message, a missed call, a notification, and the like.

An electronic device having the foregoing hardware structure in the solutions in the following embodiments may be a wearable device. A smartwatch is used as an example. Specifically, refer to FIG. 4. The smartwatch 20 includes the electrochemical sensing circuit 22 and the external interfaces 21 disposed on a back surface (as shown in FIG. 2) of the smartwatch 20. The external interfaces 21 are coupled to the electrochemical sensing circuit 22. The electrochemical sensing circuit 22 in FIG. 4 may be the electrochemical sensing circuit 22 in FIG. 6, and the external interfaces 21 in FIG. 4 may be the external interfaces 21 in FIG. 6.

When the back surface of the smartwatch 20 is worn facing the human body, after the external interfaces 21 (for example, FIG. 4 shows the three external interfaces 21-1, 21-2, and 21-3) are connected to the electrode interfaces 12 (for example, FIG. 8 shows the three electrode interfaces 12-1, 12-2, and 12-3) of the patch 10, the electrical signal generated by the patch 10 is transmitted to the electrochemical sensing circuit 22, and the electrochemical sensing circuit 22 is configured to convert the electrical signal into the detection signal. For example, the electrochemical sensing circuit 22 may specifically transmit the detection signal to a digital signal processor of the smartwatch, and the digital signal processor converts the detection signal into a display signal and outputs the display signal to a display to display a detected blood glucose value to the user. Specifically, with reference to the foregoing electrochemical sensing circuit 22 provided in FIG. 7, the blood glucose detection is used as an example. The electrical signal generated by the patch 10 may be specifically the current passing through the two electrodes WE and CE. The detection signal may be specifically the voltage eo into which the current passing through the WE and the CE is converted. The digital signal processor may perform analog-to-digital conversion on the voltage eo, to convert the voltage eo into the display signal indicating the blood glucose value (for example, 6.0 mg/dL (milligram/deciliter)) indicating blood glucose (glucose) content in the blood. The blood glucose value is displayed on the display 293 by using a display signal.

An embodiment of this application further provides a patch used with the foregoing smartwatch 20. Refer to FIG. 8. The patch 10 includes a substrate 14, the electrode interfaces 12 disposed on a first surface of the substrate 14 (the patch 10 that can implement blood glucose detection is used as an example, and includes the three electrodes; and therefore, the three electrode interfaces 12-1, 12-2, and 12-3 need to be disposed), and an adhesive layer 15 disposed on a second surface of the substrate 14, where the second surface faces away from the first surface. The patch 10 further includes the microneedle 13 disposed on the second surface of the substrate 14. The electrodes 11 are disposed on the microneedle 13 (in FIG. 8, one microneedle 13 is used as an example, where the three electrodes 11-1, 11-2, and 11-3 are disposed on the microneedle 13). The electrodes disposed on the microneedle 13 include at least the work electrode WE and the reference electrode RE. The work electrode WE is coated with a plurality of coatings for physiological parameter detection. The electrodes 11 (11-1, 11-2, and 11-3) are one-to-one coupled to the electrode interfaces 12 (12-1, 12-2, and 12-3). In FIG. 8, the three electrodes are used as an example for description. In addition to the work electrode WE and the reference electrode RE, the counter electrode CE is further included. The work electrode WE, the reference electrode RE, and the electrode CE may be any one of the electrodes 11 (11-1, 11-2, and 11-3). For example, the electrode 11-1 is the reference electrode RE, the electrode 11-2 is the counter electrode, and the electrode 11-3 is the work electrode WE. Materials of the substrate 14 include but are not limited to polyimide (polyimide, PI), polyethylene terephthalate (polyethylene terephthalate, PET), polydimethylsiloxane (polydimethylsiloxane, PDMS), polyurethane (polyurethane, PU), and the like. The adhesive layer 15 is mainly used to adhere the patch 10 to the skin to prevent the patch 10 from falling off. The adhesive layer 15 may be made of a skin-friendly material, including but not limited to silicone gels.

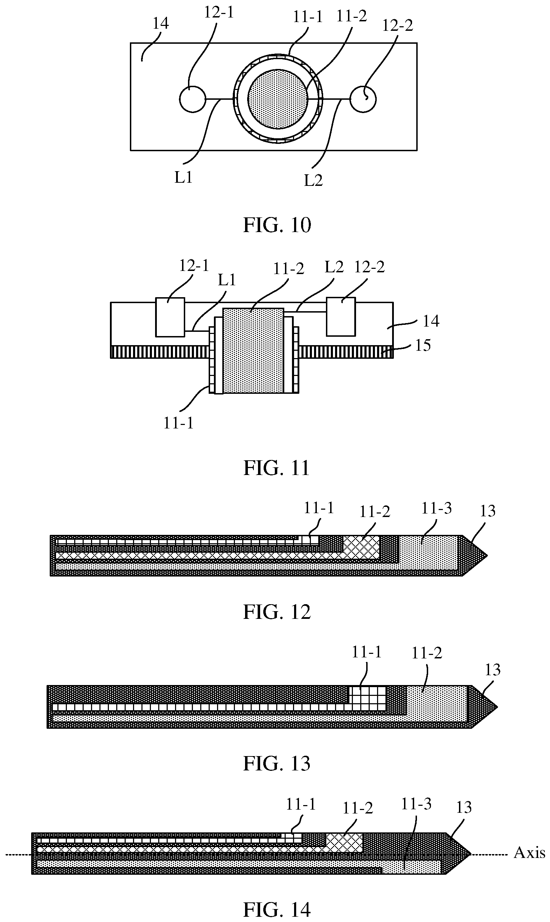

The two electrodes are used as an example for description. As shown in FIG. 9, the work electrode WE and the reference electrode RE may be any one of the electrodes 11 (11-1 and 11-2). For example, the electrode 11-1 is the reference electrode RE, and the electrode 11-2 is the work electrode WE. To explain a coupling mode between the electrodes 11 and the electrode interfaces 12, as shown in FIG. 10 and FIG. 11, the electrode 11-1 and the electrode interface 12-1 are electrically connected through a lead L1, and the electrode 11-2 and the electrode interface 12-2 are electrically connected through a lead L2. Conductive structures at both ends may be connected through the lead L1 (L2) in a bonding (bonding) mode.

In this way, when the patch 10 is applied to the human body (as shown in FIG. 5), the microneedle 13 pierces into the dermis of the human body, so that the electrodes 11 can be in contact with the blood of the dermis. When the smartwatch 20 is worn on the human body (for example, the wrist), the electrical connections between the external interfaces 21 of the smartwatch 20 and the electrode interfaces 12 of the patch are formed (for example, the electrical connection relationships are shown by the dashed lines in FIG. 2; the three-electrode patch is used as an example; and as shown in FIG. 4, for example, the external interface 21-1 and the electrode interface 12-1 are electrically connected, the external interface 21-2 and the electrode interface 12-2 are electrically connected, and the external interface 21-3 and the electrode interface 12-3 are electrically connected). The electrical signal may be transmitted between the patch 10 and the electronic device 20. In this way, the components (such as the glucose, the oxygen, and the blood lipids) in the blood generate the electrochemical reactions at the electrodes to form the electrical signals. For example, for the blood glucose detection, the glucose in the blood generates the electrochemical reaction on the coating coated on the work electrode WE of the electrode interfaces 12 to form the electrical signal. In this way, the electrochemical sensing circuit 22 can detect the electrical signal formed through the electrochemical reaction, and the electrical signal is further converted into the detection signal to detect the physiological parameter. In this way, because no circuit module such as the electrochemical sensing circuit, the data processing module, and the transmission module is disposed on the patch 10, the structure of the patch 10 is simplified, and the use costs are reduced.

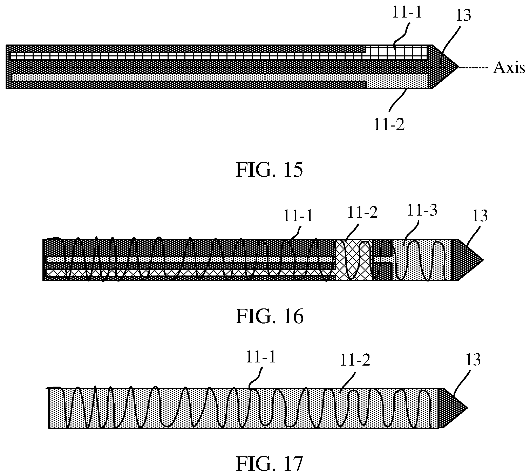

Shapes of the electrodes 11 include a columnar shape, a planar shape, or a wire-wound shape. The three-electrode patch 10 is used as an example. Refer to FIG. 12. The electrode 11-1, the electrode 11-2, and the electrode 11-3 may be three planar conductive structures that are sequentially arranged on the microneedle 13. The two-electrode patch 10 is used as an example. Refer to FIG. 13. The electrode 11-1 and the electrode 11-2 may be two planar conductive structures that are sequentially arranged on the microneedle 13. As shown in FIG. 14 and FIG. 15, the planar electrodes may be separately disposed in two opposite directions along an axis of the microneedle to increase a contact area between the electrodes 11 and the dermis of the skin. The three-electrode patch 10 is used as an example. As shown in FIG. 14, the electrode 11-1 and the electrode 11-2 are disposed on a same side along the axis of the microneedle, and the electrode 11-3 is disposed on the other side along the axis of the microneedle. The two-electrode patch 10 is used as an example. As shown in FIG. 15, the electrode 11-1 and the electrode 11-2 are respectively disposed on both sides along the axis of the microneedle. The electrodes may alternatively be disposed in the columnar shape or the wire-wound shape to increase the contact area between the electrodes and the dermis layer of the skin. As shown in FIG. 8, the three-electrode patch 10 is used as an example. The electrode 11-1, the electrode 11-2, and the electrode 11-3 may be formed into three columnar conductive structures sequentially arranged on the microneedle 13. As shown in FIG. 8, diameters of the electrodes are different. For example, the diameter of the electrode 11-1 is larger than the diameter of the electrode 11-2, and the diameter of the electrode 11-2 is larger than the diameter of the electrode 11-3. As shown in FIG. 9, the two-electrode patch 10 is used as an example. The electrode 11-1 and the electrode 11-2 may be formed into two columnar conductive structures sequentially arranged on the microneedle 13. As shown in FIG. 9, diameters of the electrodes are different. For example, the diameter of the electrode 11-1 is larger than the diameter of the electrode 11-2. As shown in FIG. 16, the three-electrode patch 10 is used as an example, the electrode 11-1 may be wound around the microneedle 13 by using a wire-wound conductive structure, and shapes of the other two electrodes are not limited. However, it should be noted that the wire-wound electrode 11-1 needs to be insulated from the electrode 11-2 and the electrode 11-3. Similarly, as shown in FIG. 17, the two-electrode patch 10 is used as an example, the electrode 11-1 may be wound around the microneedle 13 by using a wire-wound conductive structure, and a shape of the other one electrode is not limited. However, it should be noted that the wire-wound electrode 11-1 needs to be insulated from the electrode 11-2.

In addition, materials of the electrodes include but are not limited to one or more of the following materials: lamp black carbon (lamp black carbon), glassy carbon, graphite, silver, silver chloride, platinum, palladium, a platinum-iridium alloy, titanium, gold, and iridium. In particular, the reference electrode RE may use the silver or the silver chloride. Since a silver or silver chloride electrode has little solubility, and extremely high stability and reversibility in an aqueous solution system, and a surface of the electrode is well protected even hydrogen exists, when the silver or silver chloride electrode is used as the reference electrode, self-noise of the electrode can be minimized.

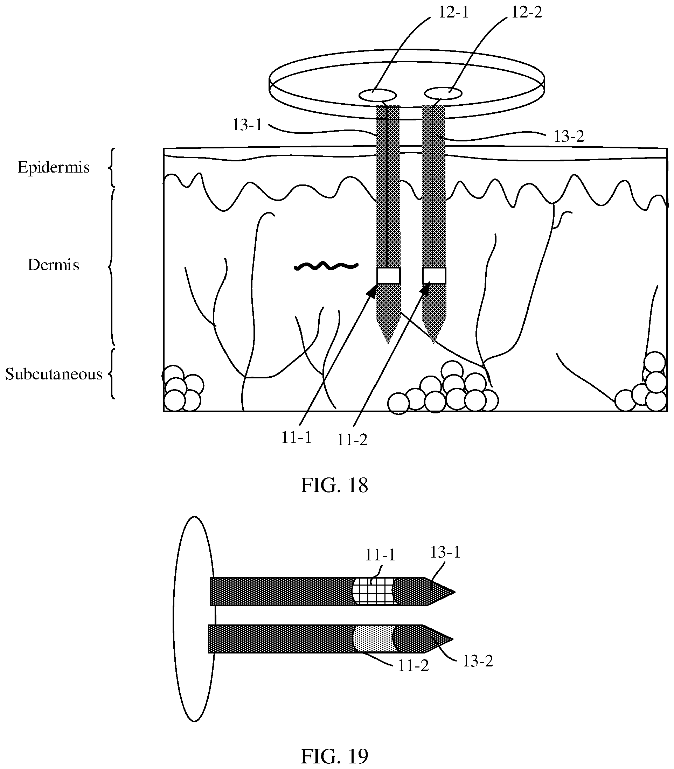

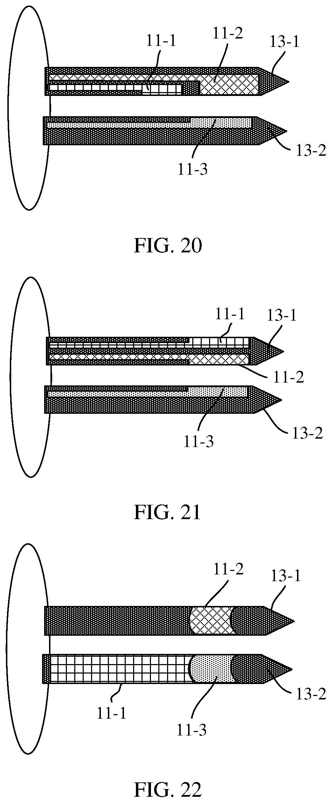

The foregoing is mainly described by using an example in which the patch 10 has one microneedle. In addition, the patch 10 may also have two or more microneedles. As shown in FIG. 18, the patch 10 has two microneedles 13-1 and 13-2. With reference to FIG. 19 to FIG. 22, a manner in which the electrodes of the two-electrode patch 10 and the electrodes of the three-electrode patch 10 are disposed on two microneedles is further provided. Specifically, refer to FIG. 19. The two-electrode patch 10 is used as an example. The electrode 11-1 may be disposed on the microneedle 13-1, and the electrode 11-2 may be disposed on the microneedle 13-2. For example, shapes of the electrodes in FIG. 19 are the columnar shape. As described above, the shapes of the electrodes may alternatively be another shape. Refer to FIG. 20 to FIG. 22. The three-electrode patch 10 is used as an example. The electrode 11-1 and the electrode 11-2 may be disposed on the microneedle 13-1, and the electrode 11-3 may be disposed on the microneedle 13-2. Alternatively, the electrode 11-1 and the electrode 11-3 may be disposed on the microneedle 13-2, and the electrode 11-2 may be disposed on the microneedle 13-1, and the like. It may be understood that there is another form of disposing the three electrodes on the two microneedles. This is not enumerated herein. In addition, in FIG. 20 and FIG. 21, planar electrodes are mainly used as an example. In FIG. 22, columnar electrodes are mainly used as an example for description. As described above, the shapes of the electrodes may alternatively be another shape.

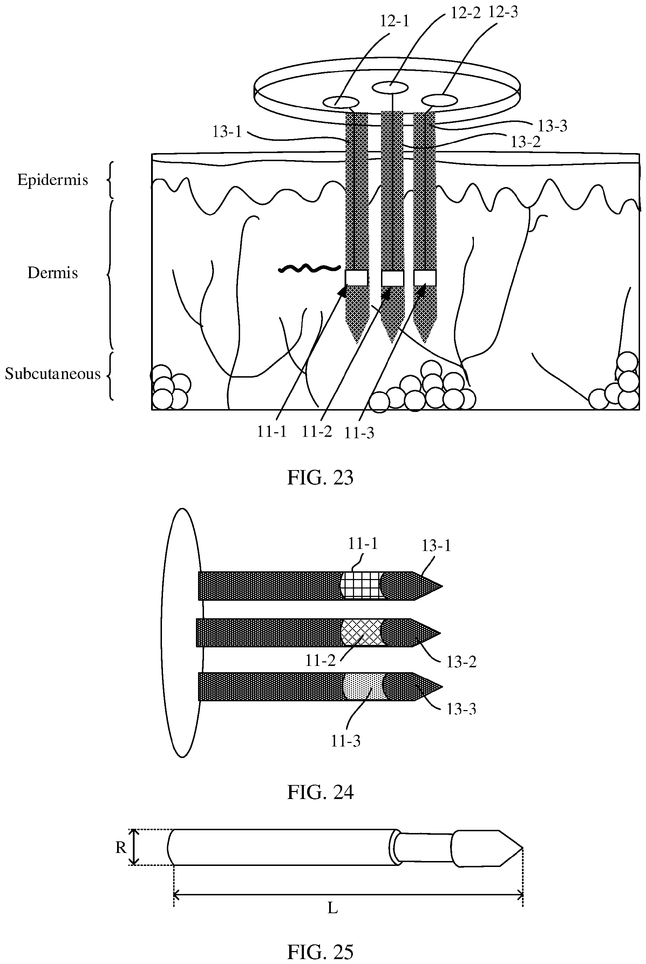

As shown in FIG. 23, the patch 10 has the three microneedles (13-1, 13-2, and 13-3). With reference to FIG. 24, the three-electrode patch 10 is used as an example. The electrode 11-1 may be disposed on the microneedle 13-1, the electrode 11-2 may be disposed on the microneedle 13-2, and the electrode 11-3 may be disposed on the microneedle 13-2. For example, shapes of the electrodes in FIG. 24 are the columnar shape. As described above, the shapes of the electrodes may alternatively be another shape.

When the patch 10 is applied to the skin, it is necessary to implant, by piercing into the epidermis, the microneedle into the dermis of the upper part of subcutaneous tissue to ensure effective contact of the electrodes with the blood. An implantation manner may be an active pressing manner. For example, the user manually applies the patch 10 to the back of the wrist, and uses the microneedle to pierce into the subcutaneous tissue. Alternatively, a manner in which a needle aid is used to assist implantation is used.

To ensure fast implantation, less pain, a small wound surface, and that a length of the microneedle is sufficient for subcutaneous placement, as shown in FIG. 25, a length L of the microneedle is from 1 to 5 mm, and a diameter R of the microneedle is from 7 to 400 µm. In addition, in the above example, a material of the microneedle 13 includes but is not limited to stainless steel, platinum, or a polymer material. When the active pressing manner is used in combination with the two implantation manners of the microneedle, the microneedle usually uses a rigid material such as the stainless steel due to different speeds of action of users. In addition, to ensure requirements of fast implantation, less pain, and a small wound surface, a shape of a needle tip of the microneedle may be designed to be a pine needle shape. When the manner in which the needle aid is used to assist implantation is used, because the needle aid may have a high implantation speed, a needle body may use a flexible material such as the polymer material or the platinum. Because the needle aid may have the high implantation speed, the needle tip of the microneedle does not need to be specially designed.

In addition, the external interfaces 21 may be disposed as the magnetic interfaces or may use the magnetic material, and the electrode interfaces 12 may be disposed as the magnetic interfaces or may use the magnetic material. For example, the external interfaces 21 and the electrode interfaces 12 may use magnetic materials such as neodymium iron boron, samarium cobalt, aluminum nickel cobalt, and iron chromium cobalt, and the magnetic materials may be magnetized by using a magnetic field, so that the magnetic materials have magnetism. The external interfaces 21 and the electrode interfaces 12 may be magnetically engaged to ensure the reliability of the electrical connections between the external interfaces 21 and the electrode interfaces 12. Specifically, both the external interfaces 21 and the electrode interfaces 12 may have the magnetism (for example, opposite magnetism). Alternatively, one has the magnetism, and the other is the magnetic material. In addition, when both the external interfaces 21 and the electrode interfaces 12 use the magnetic materials, one or more layers of materials with high conductivity such as nickel and copper may be manufactured, through electroplating or metal deposition, on surfaces on which the external interfaces 21 and the electrode interfaces 12 are in contact with each other, to increase conductivity of the external interfaces 21 and the electrode interfaces 12. In this way, because the external interfaces 21 and the electrode interfaces 12 are engaged in a weak magnetic mode, the reliability of the electrical connections between the external interfaces 21 and the electrode interfaces 12 can be ensured. When the external force disturbs the electronic device 20, the external interfaces 21 and the electrode interfaces 12 may be detached to avoid the case in which the disturbance of the electronic device 20 causes the patch 10 to scratch the human skin.

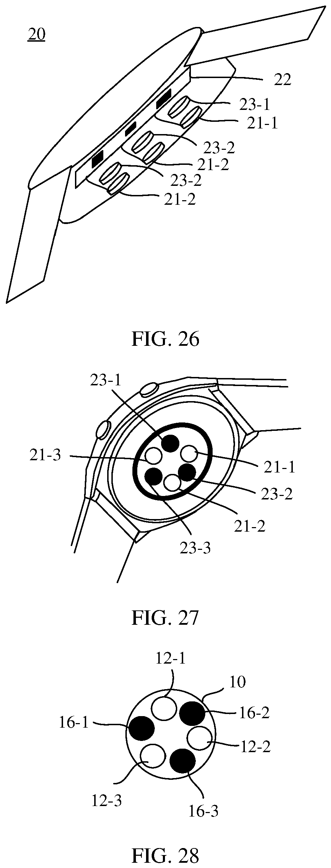

Certainly, the magnetic interfaces may also be separately disposed on the smartwatch 20 and the patch 10 to engage the smartwatch 20 with the patch 10. Specifically, as shown in FIG. 26, second magnetic interfaces 23 that one-to-one correspond to the external interfaces 21 may be disposed inside the smartwatch 20 and on inner sides of the external interfaces 21 (for example, the three-electrode patch 10 scenario is used as an example, a second magnetic interface 23-1 is disposed on an inner side of an external interface 21-1, a second magnetic interface 23-2 is disposed on an inner side of an external interface 21-2, and a second magnetic interface 23-3 is disposed on an inner side of an external interface 21-3). The second magnetic interfaces 23 have the magnetism, or the second magnetic interfaces 23 are the magnetic materials. In this way, when the electrode interfaces 12 are disposed as the magnetic interfaces or use the magnetic materials, the electronic device 20 and the patch 10 may be engaged through the second magnetic interfaces 23 to ensure the reliability of the electrical connections between the external interfaces 21 and the electrode interfaces 12.

In addition, refer to FIG. 27 and FIG. 28. Alternatively, first magnetic interfaces 16 may be disposed on the first surface of the substrate of the patch 10. The first magnetic interfaces 16 have the magnetism, or the first magnetic interfaces 16 are the magnetic materials. The second magnetic interfaces 23 are disposed on the smartwatch 20. The second magnetic interfaces 23 have the magnetism, or the second magnetic interfaces 23 are the magnetic materials. One surface of the second magnetic interfaces 23 exposes the electronic device 20. Specifically, both the first magnetic interfaces 16 and the second magnetic interfaces 23 may have the magnetism (for example, the opposite magnetism). Alternatively, one has the magnetism, and the other is the magnetic material. Positions of the first magnetic interfaces 16 one-to-one correspond to positions of the second magnetic interfaces 23. After the smartwatch 20 and the patch 10 are attached through the first magnetic interfaces 16 and the second magnetic interfaces 23, the external interfaces 21 and the electrode interfaces 12 are electrically connected. Specifically, the three-electrode patch 10 scenario is used as an example. A position of a first magnetic interface 16-1 corresponds to a position of the second magnetic interface 23-1, and the first magnetic interface 16-1 and the second magnetic interface 23-1 are engaged. A position of a first magnetic interface 16-2 corresponds to a position of the second magnetic interface 23-2, and the first magnetic interface 16-2 and the second magnetic interface 23-2 are engaged. A position of a first magnetic interface 16-3 corresponds to a position of the second magnetic interface 23-3, and the first magnetic interface 16-3 and the second magnetic interface 23-3 are engaged. Certainly, the foregoing uses an example in which the smartwatch 20 includes the three second magnetic interfaces 23 and the patch 10 includes the three first magnetic interfaces 16 for description. Because fixing two points on one plane can ensure that the plane cannot rotate along an axis perpendicular to the plane, at least two second magnetic interfaces 23 with different positions need to be disposed on the smartwatch 20, and at least two first magnetic interfaces 16 with different positions need to be disposed on the patch 10, to ensure the reliability of the electrical connections between the external interfaces 21 and the electrode interfaces 12, in other words, to prevent a case in which the smartwatch rotates along an axis perpendicular to a display plane of the smartwatch, causing the external interfaces 21 and the electrode interfaces 12 to detach.

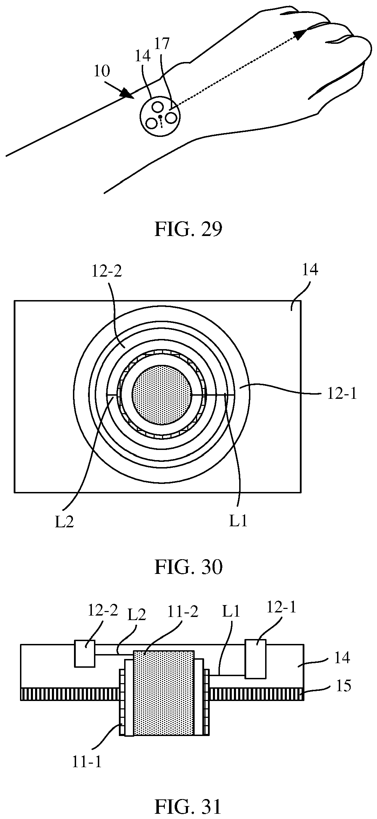

In addition, that the external interfaces 21 of the smartwatch 20 and the electrode interfaces 12 of the patch 10 form correct connection modes needs to be ensured. For example, the three-electrode patch is used as an example. It needs to be ensured that after the patch is applied to the skin, the external interface 21-1 of the smartwatch and the electrode interface 12-1 of the patch are electrically connected, the external interface 21-2 and the electrode interface 12-2 are electrically connected, and the external interface 21-3 and the electrode interface 12-3 are electrically connected. An application mark 17 is disposed on the first surface of the substrate 14, and the application mark 17 indicates an application direction of the patch. As shown in FIG. 29, when the patch is applied to the skin, the application mark 17 indicates application in a direction between the ring finger and the middle finger.

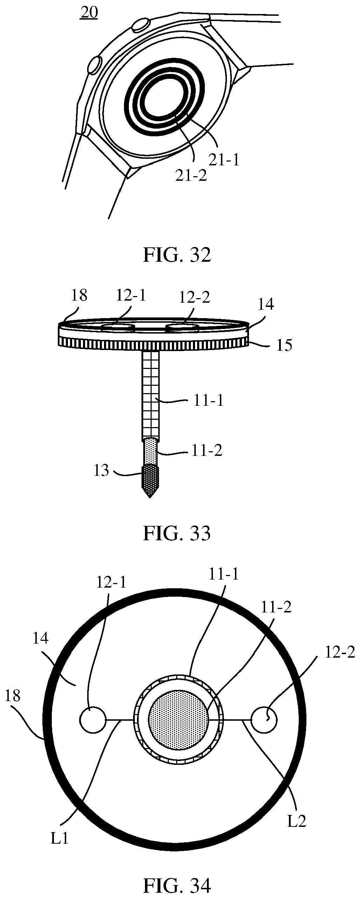

In another example, the electrode interfaces of the patch are concentrically disposed ring-shaped electrode interfaces. Corresponding external interfaces of the smartwatch are concentrically disposed ring-shaped external interfaces. The two-electrode patch is used as an example. Refer to FIG. 30 and FIG. 31. The electrode interface 12-1 and the electrode interface 12-2 may be concentrically disposed ring-shaped electrode interfaces. The electrode interface 12-1 is coupled to the electrode 11-1 through the lead L1, and the electrode interface 12-2 is coupled to the electrode 11-2 through the lead L2. As shown in FIG. 32, the external interface 21-1 and the external interface 21-1 may be concentrically disposed ring-shaped external interfaces. After the smartwatch 20 and the patch 10 are attached, the electrode interface 12-1 and the external interface 21-1 are electrically connected, and the electrode interface 12-2 and the external interface 21-2 are electrically connected.

In another example, the patch further includes a water retaining strip that is disposed on the first surface of the substrate, and the water retaining strip surrounds the electrode interfaces. As shown in FIG. 33 and FIG. 34, the water retaining strip 18 is disposed on the first surface of the substrate 14, and the water retaining strip 18 is disposed around at least two electrode interfaces 12 by surrounding the at least two electrode interfaces 12. In this way, when the water retaining strip 18 and the smartwatch 20 are attached, closed space may be formed between the back surface of the smartwatch 20 and the substrate 14 to avoid impact of conductive liquid such as water or sweat stains on the electrode interfaces. For example, when a plurality of electrode interfaces are disposed on the patch, the conductive liquid may short-circuit the plurality of electrode interfaces, thereby affecting reliability of physiological parameter detection. Certainly, in the figure, the water retaining strip 18 is shown by using a ring shape as an example. It may be understood that a shape of the water retaining strip 18 is not required in this application, for example, a closed pattern of another ring shape. Certainly, in some examples, the water retaining strip may alternatively be directly disposed on the back surface of the smartwatch 20, and there is no need to dispose the water retaining strip on each patch, thereby further reducing the costs.

In addition, in some examples, various different membrane layers need to be modified on a surface of the work electrode of the microneedle to achieve effective physiological parameter detection. The glucose detection (that is, the blood glucose detection) is used as an example. To successfully convert a chemical signal of the glucose into an electrical signal, the following lists membrane layers that may appear on the surface of the work electrode and corresponding functions.

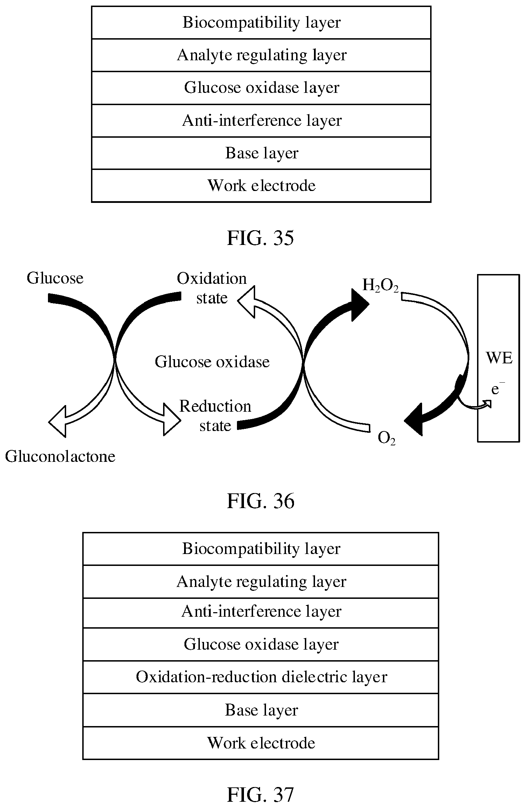

Refer to FIG. 35. A base layer, an anti-interference layer, a glucose oxidase layer, an analyte regulating layer, and a biocompatibility layer are sequentially stacked on the work electrode. A function of the biocompatibility layer is improving affinity between the patch and tissue in the body, reducing pollution caused by immunity and protein attachment in the body, and increasing effective use time of the patch. Common materials used for the biocompatibility layer include but are not limited to hydrogels, polylactic acid-hydroxyethyl copolymers, and the like. The analyte regulating layer is also referred to as a glucose restriction layer. Usually, the body has a high concentration of glucose, but the work electrode has a very small amount of glucose oxidase and a very small amount of oxygen. To ensure that the glucose oxidase is excessive, but the glucose is not excessive, the glucose that penetrates the membrane needs to be limited, and a function of the analyte regulating layer is limiting a proportion of the glucose inside and outside the membrane. A ratio usually ranges from 1:1 to tens of thousands to one. Common materials used for the analyte regulating layer include but are not limited to vinylpyridine-polydiethanol copolymers, PU, poly2-hydroxyethyl methacrylate, and the like. The glucose oxidase layer including the glucose oxidase (a systematic name is β-D-glucose oxidoreductase, and an English full name is glucose oxidase, where GOx or GOD is for short) performs modification through covalent crosslinking or dehydration condensation, and is used for catalytic oxidation of the glucose. For the anti-interference layer in this example, the glucose reacts with the oxygen at the glucose oxidase layer to generate hydrogen peroxide. However, various interfering substances in human tissue fluid may interfere with the blood glucose detection of the patch. The anti-interference layer allows, by filtering out the interfering substances, only the hydrogen peroxide (H₂O₂) to pass through, to avoid or reduce the interference. Commonly used materials include but are not limited to a perfluorosulfonic acid membrane (nafion). A function of the base layer is modifying the surface of the work electrode, so that coatings at the base layer can be better attached to the surface of the work electrode and are more firmly connected. Common materials used for the base layer include but are not limited to polyvinylpyridine derivatives, chitosan, glutaraldehyde, and the like.

Refer to FIG. 36 and the following chemical formulas 1, 2, 3, and 4. A working principle of the work electrode having a coating structure provided in FIG. 35 is explained as follows: The oxygen O₂ dissolved in the subcutaneous tissue fluid participates in a glucose (glucose) oxidation process, and passes through the biocompatibility layer and the analyte regulating layer together with the glucose to the glucose oxidase layer to generate a reaction. First, the oxidation of the glucose (glucose) is catalyzed by the glucose oxidase (GOx) to generate gluconolactone (gluconolactone), and the glucose oxidase is changed from an oxidation state to a reduction state (as shown in the formula 1). In the formula 1, after flavin adenine dinucleotide (FAD) in the oxidation state is used as an oxidant to provide the oxygen O₂, and is then reduced to flavin adenine dinucleotide (FADH₂) in the reduction state, the glucose oxidase in the reduction state is oxidized by the oxygen O₂ to the hydrogen peroxide (H₂O₂) (as shown in the formula 2). In the formula 2, the flavin adenine dinucleotide (FADH₂) in the reduction state is oxidized by the oxygen O₂ to the flavin adenine dinucleotide (FAD) in the oxidation state. The formula 1 and the formula 2 are equivalent to the formula 3. To be specific, the glucose (glucose) and the oxygen O₂ are catalyzed by the glucose oxidase GOx to generate the gluconolactone (gluconolactone) and the hydrogen peroxide (H₂O₂). Finally, the hydrogen peroxide (H₂O₂) passes through the anti-interference layer and reaches the base layer, and undergoes an oxidation reaction or a reduction reaction under an action of a voltage (from 0.6 to 0.7 V) of the work electrode to generate a current e".

Coatings of the work electrode include but are not limited to the foregoing base layer, anti-interference layer, enzyme sensing layer, analyte regulating layer (semipermeable membrane), biocompatibility layer, and the like. A sequence of the coatings may be slightly adjusted (for example, the anti-interference layer may be disposed between the analyte regulating layer and the biocompatibility layer), or some coatings may be mixed into one layer for use.

GOx (FAD)+glucose → GOx (FADH₂)+gluconolactone formula 1

GOx (FADH₂)+oxidant (O₂) → GOx (FAD)+product (H₂O₂) formula 2

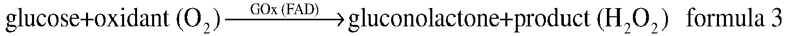

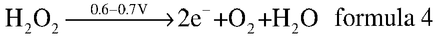

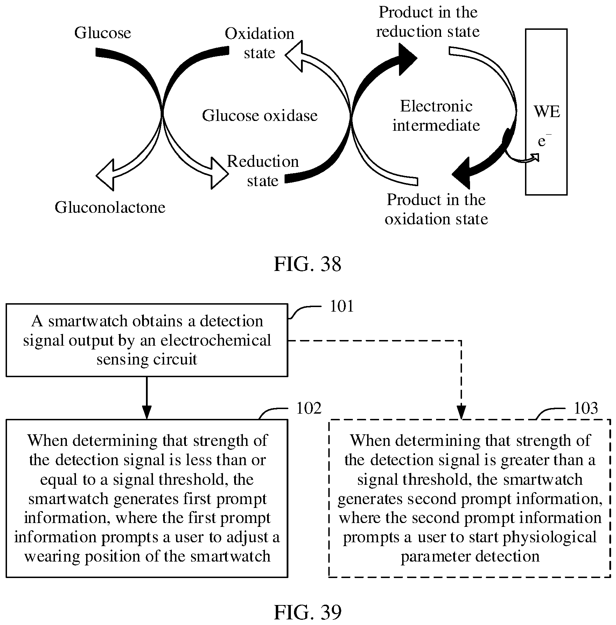

In another solution, as shown in FIG. 37, a base layer, an oxidation-reduction dielectric layer, a glucose oxidase layer, an anti-interference layer, an analyte regulating layer, and a biocompatibility layer are sequentially stacked on the work electrode WE. For functions and materials of other layers other than the oxidation-reduction dielectric layer, refer to descriptions in the embodiment corresponding to FIG. 35. The oxidation-reduction dielectric layer is a polymer chain material based on an osmium complex (Os). A function of the oxidation-reduction dielectric layer is transmitting electrons at low potential, and reducing influence of interfering substances (ascorbic acid, uric acid, and the like). Refer to FIG. 38 and the following chemical formulas 5, 6, 7, and 8. A working principle of the work electrode having a coating structure provided in FIG. 37 is explained as follows: A difference from the solution provided in FIG. 35 is that the oxygen O₂ does not participate in an oxidation-reduction reaction, and the oxidation-reduction dielectric layer replaces the oxygen. First, the glucose (glucose) passes through the biocompatibility layer, the analyte regulating layer, and the anti-interference layer to the glucose oxidase layer, and the oxidation of the glucose is catalyzed by oxidized glucose oxidase (GOx) to generate gluconolactone (gluconolactone). The oxidized glucose oxidase is reduced to reduced glucose oxidase (GOx) (as shown in the formula 5). In the formula 5, after flavin adenine dinucleotide (FAD) in an oxidation state is used as an oxidant to provide the oxygen O₂, and is then reduced to flavin adenine dinucleotide (FADH₂) in a reduction state, the reduced glucose oxidase is oxidized by an electronic intermediate (for example, the osmium complex Os) in the oxidation state, and the electronic intermediate in the oxidation state changes to a product (as shown in the formula 6) in the reduction state. In the formula 6, the flavin adenine dinucleotide (FADH₂) in the reduction state is oxidized by the electronic intermediate in the oxidation state to the flavin adenine dinucleotide (FAD) in the oxidation state. The formula 5 and the formula 6 are equivalent to the formula 7. To be specific, the glucose (glucose) and the electronic intermediate (for example, the osmium complex Os) in the oxidation state are catalyzed by the glucose oxidase GOx to generate the gluconolactone (gluconolactone) and an electronic intermediate in the reduction state. Finally, a voltage is applied to the work electrode WE (because the oxidation-reduction dielectric layer is used, the oxygen does not participate in the reaction, and no hydrogen peroxide is generated, electron transfer can be completed by applying a small voltage (0.2 V) to the work electrode), and the product in the reduction state is oxidized for another time to generate a current corresponding to a glucose concentration.

In this solution, coatings of the work electrode include but are not limited to the base layer, oxidation-reduction dielectric layer, enzyme sensing layer, anti-interference layer, analyte regulating layer (semipermeable membrane), biocompatibility layer, and the like. A sequence of the coatings may be slightly adjusted (for example, the anti-interference layer may be disposed between the biocompatibility layer and the analyte regulating layer), or some coatings may be mixed into one layer for use. In this embodiment, since the oxygen does not participate in the reaction, the interfering substance causes little interference to the blood glucose detection of the patch, and therefore the interference layer may be omitted.

GOx (FAD)+glucose → GOx (FADH₂)+gluconolactone formula 5

GOx (FADH₂)+Os (oxidation state) → GOx (FAD)+Os (reduction state) formula 6

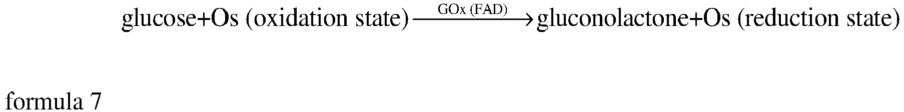

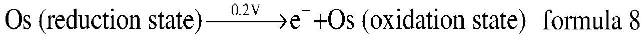

Based on the foregoing smartwatch and patch, after the smartwatch is worn on a part of the human body, movement of the human body causes slight disturbance to the smartwatch. In this way, when the disturbance of the smartwatch causes the external interfaces of the smartwatch and the electrode interfaces of the patch to disconnect, normal execution of the physiological parameter detection may be affected. Therefore, to ensure that the external interfaces 21 of the smartwatch 20 and the electrode interfaces 12 of the patch 10 are electrically connected when the smartwatch is worn on the human body, to ensure that physiological parameter detection is normally performed, an embodiment of this application provides a patch in-position detection method. Refer to FIG. 39, the method specifically includes the following steps.

101: A smartwatch obtains a detection signal output by an electrochemical sensing circuit.

The detection signal may be a signal, such as a voltage or a current, with specific signal strength.

102: When determining that the strength of the detection signal is less than or equal to a signal threshold, the smartwatch generates first prompt information, where the first prompt information prompts a user to adjust a wearing position of the smartwatch.

103: When determining that the strength of the detection signal is greater than the signal threshold, the smartwatch generates second prompt information, where the second prompt information prompts the user to start physiological parameter detection.

The first prompt information and the second prompt information include vibration, a sound, or display information.

The detection signal output by the electrochemical sensing circuit may reflect connection statuses between the external interfaces 21 of the smartwatch 20 and the electrode interfaces 12 of the patch 10. For example, if the strength of the detection signal is zero, it indicates that the external interfaces 21 of the smartwatch 20 and the electrode interfaces 12 of the patch 10 are completely disconnected. Alternatively, if the detection signal output is greater than 0 but less than the signal threshold, it indicates that the external interfaces 21 of the smartwatch 20 are in poor contact with the electrode interfaces 12 of the patch 10, and a high impedance contact problem exists. In this case, a wearing posture of the smartwatch 20 needs to be adjusted, so that the external interfaces 21 of the smartwatch 20 and the electrode interfaces 12 of the patch 10 are electrically reconnected.

Specifically, a complete blood glucose detection process is used as an example. A process of using the smartwatch 20 and the patch 10 provided in embodiments of this application is described as follows: First, the user attaches the patch 10 to the back of the wrist in the active pressing manner or the manner in which the needle aid is used to assist implantation, and then wears the smartwatch 20. After the smartwatch 20 is correctly worn, the external interfaces 21 of the smartwatch 20 and the electrode interfaces 12 of the patch 10 are electrically connected. Then, a blood glucose detection function is manually or automatically enabled.

For example, the smartwatch 20 may display a first interface, and receive a first operation performed by the user on the first interface. The first operation is used to trigger the smartwatch 20 to perform blood glucose detection. Specifically, the smartwatch 20 may display a first interface of a first APP. The first interface may be used to trigger the smartwatch 20 to perform blood glucose detection. For example, the first interface may display a trigger identifier of "detect blood glucose", to indicate that the user may trigger blood glucose detection by tapping the identifier of "detect blood glucose". In addition, a blood glucose detection mode, for example, a single detection mode or a continuous detection mode, may be further triggered. In the continuous detection mode, parameters such as a detection time interval may be further configured.

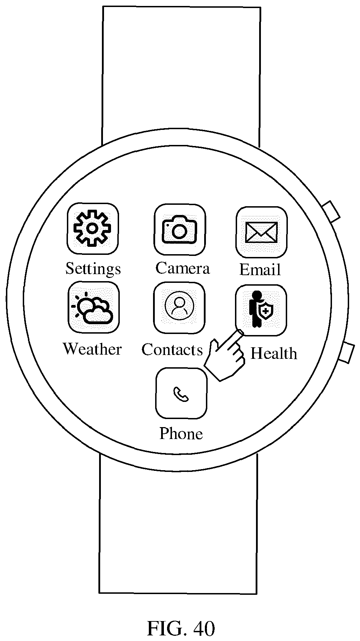

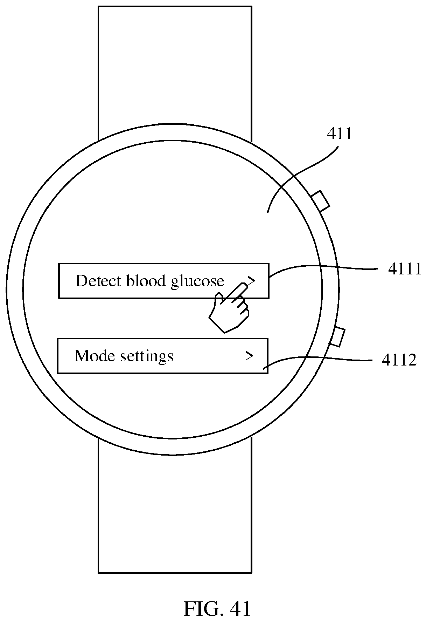

For example, the first APP in this embodiment of this application may be an application corresponding to an icon "health" shown in FIG. 40. As shown in FIG. 40, the smartwatch 20 may receive a tap operation performed by the user on the icon "health". In response to the tap operation, the smartwatch 20 may start the first APP, and display a home page (that is, a first interface 411) of the first APP in FIG. 41. The first interface 411 may include a health management option, for example, a "detect blood glucose" option (a control 4111 on the first interface 411). The control 4111 is used to trigger the smartwatch to detect the blood glucose. The first operation may be a tap operation (for example, a single tap operation) performed by the user on the control 4111 shown in the first interface 411, for example, the tap operation on the control 4111. Alternatively, the first operation may further be a preset gesture input by the user on the first interface 411 (as shown in FIG. 41), for example, an S-shaped gesture, an L-shaped gesture, a dual-point up-sliding gesture with two fingers, or a three-point up-sliding gesture with three fingers. In addition, the smartwatch 20 may prompt the user on the first interface of the preset gesture and a function (that is, the blood glucose detection) triggered by the preset gesture.

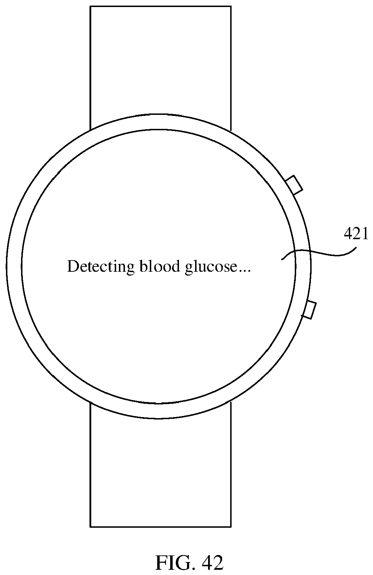

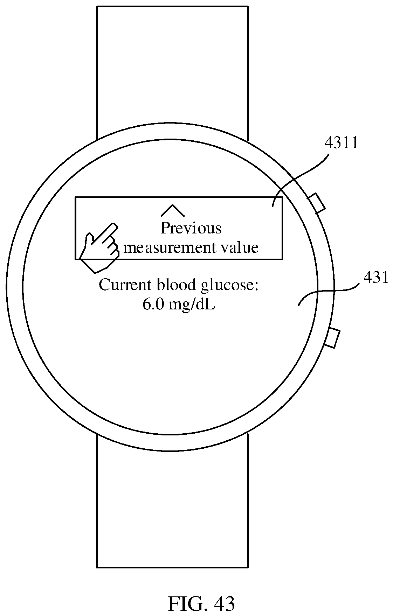

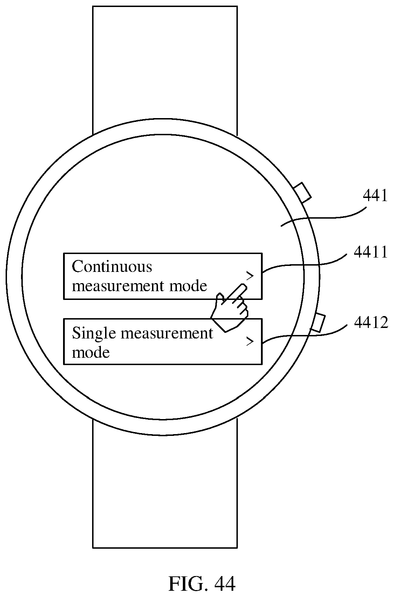

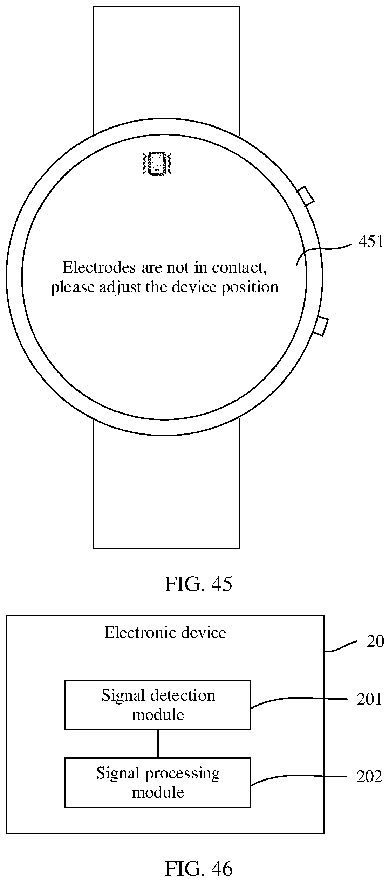

Specifically, when the user taps the control 4111, the smartwatch 20 displays a second interface 421 in response to the tap operation performed by the user on the control 4111 (as shown in FIG. 42, "detecting blood glucose" is displayed on the second interface 421 to prompt the user that the smartwatch 20 is performing blood glucose detection). In a process in which the smartwatch 20 displays the second interface 421, the smartwatch 20 applies a constant voltage between the work electrode and the reference electrode of the patch 10, to start formal blood glucose detection. In this case, the oxidation of the glucose may be catalyzed by the coating of the work electrode to generate the electrical signal. The electrochemical sensing circuit of the watch may capture the electrical signal, and convert the electrical signal into the detection signal. Finally, a processor of the smartwatch 20 performs signal processing and analysis on the detection signal by using an algorithm, and converts the detection signal into a glucose concentration (a blood glucose value). In addition, after the smartwatch 20 displays the second interface 421, the patch 10 may be further initialized first (in a period of time (ranging from one hour to a few hours) when the microneedle is first inserted into the body, a surface membrane layer is filled with water, which is also referred to as a hydration process, and then the surface membrane layer becomes stable, where the process is initialization of the patch 10). A calibration algorithm is executed, or the user is reminded to perform calibration by using blood glucose of a fingertip. After a measurement result is generated, the smartwatch 20 displays a third interface 431 (as shown in FIG. 43, "current blood glucose: 6.0 mg/dL" is displayed on the third interface 431). Certainly, the third interface 431 may further provide an option that prompts the user to trigger querying a "previous measurement value" (a control 4311) by using a tap operation. In addition, the first interface 411 may further include an option such as "mode settings" (a control 4112). For the control 4112 on the first interface 411 shown in FIG. 41, after the user performs a tap operation on the control 4112, the smartwatch 20 displays the fourth interface 441 (as shown in FIG. 44). An option that prompts the user to trigger the "continuous detection mode" (a control 4411) or the "single detection mode" (a control 4412) may be provided on the fourth interface 441. In response to a tap operation on the control 4411 or the control 4412, the smartwatch 20 may enter the corresponding detection mode. For example, in response to the tap operation of the control 4412, the smartwatch 20 enters the single detection mode, and completes single blood glucose measurement. In response to the tap operation of the control 4411, the smartwatch 20 enters the continuous detection mode, and may continuously measure the blood glucose at a time interval configured by the user. The foregoing provides the complete blood glucose detection process with reference to a specific example. In an actual use process, after the smartwatch is worn on the part of the human body, the movement of the human body causes slight disturbance to the smartwatch. In this way, when the disturbance of the smartwatch causes the external interfaces of the smartwatch and the electrode interfaces of the patch to disconnect, normal execution of the blood glucose detection may be affected. Therefore, after the user taps the control 4111 on the first interface 411, the smartwatch obtains the detection signal output by the electrochemical sensing circuit. For example, the detection signal may be a current signal. When it is determined that current intensity of the detection signal is less than or equal to the signal threshold (for example, the current intensity is zero or less than the signal threshold), the first prompt information is generated. For example, the first prompt information may be displaying a fifth interface 451 to the user (as shown in FIG. 45, specific display content of the fifth interface 451 may be "Electrodes are not in contact, please adjust the device position"). Certainly, the first prompt information may alternatively be vibration generated by driving the vibration motor 291, a voice prompt generated by the speaker 270A, any two of the three, or a combination of the three. In this way, after the user adjusts the position based on the prompt, the smartwatch continuously obtains the detection signal output by the electrochemical sensing circuit, and generates the second prompt information when determining that the strength of the detection signal is greater than the signal threshold. The second prompt information prompts the user to start blood glucose detection. For example, the second prompt information may directly reuse the second interface 421 (as shown in FIG. 42). Certainly, a voice or vibration prompt may also be provided at the same time.

For example, FIG. 46 is a schematic diagram of a structure of an electronic device according to an embodiment of this application. The electronic device may specifically implement the method shown in FIG. 39.

As shown in FIG. 46, the electronic device 20 includes a signal detection module 201 and a signal processing module 202. The foregoing modules or components can communicate with each other through one or more communication buses (I2C) or signal cables. A person skilled in the art may understand that the modules or components shown in FIG. 46 does not constitute a limitation on the electronic device. The electronic device 20 may include more or fewer components than those shown in the figure, or combine some components, or have different component arrangements.

The signal detection module 201 is configured to determine a connection status of a patch based on a detection signal output by an electrochemical sensing circuit. The signal processing module 202 is configured to: when determining that strength of the detection signal obtained by the signal detection module 201 is less than or equal to a signal threshold, generate first prompt information, where the first prompt information prompts a user to adjust a wearing position of the electronic device.

Optionally, the signal processing module 202 is further configured to: when determining that the strength of the detection signal is greater than the signal threshold, generate second prompt information, where the second prompt information prompts the user to start physiological parameter detection. The first prompt information and the second prompt information include vibration, a sound, or display information.

Some other embodiments of this application provide an electronic device. The electronic device may include the display (such as a touchscreen), a memory, and one or more processors. The display and the memory are coupled to the processor. The memory is configured to store computer program code. The computer program code includes computer instructions. When the processor executes the computer instructions, the electronic device may perform functions or steps performed by the electronic device in the foregoing method embodiments. For a structure of the electronic device, refer to the structure of the electronic device 20 shown in FIG. 2.

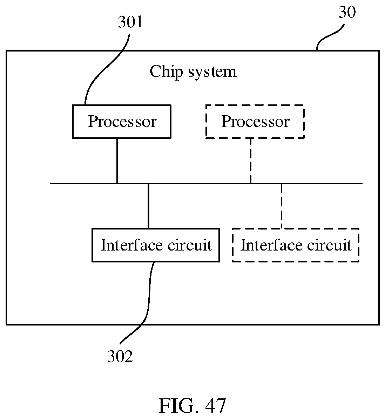

An embodiment of this application further provides a chip system. As shown in FIG. 47, the chip system 30 includes at least one processor 301 and at least one interface circuit 302. The processor 301 and the interface circuit 302 may be interconnected through a line. For example, the interface circuit 302 may be configured to receive a signal from another apparatus (for example, a memory of an electronic device). For another example, the interface circuit 302 may be configured to send a signal to another apparatus (for example, the processor 301). For example, the interface circuit 302 may read instructions stored in the memory, and send the instructions to the processor 301. When the instructions are executed by the processor 301, the electronic device is enabled to perform the steps in the foregoing embodiments. Certainly, the chip system may further include another discrete device. This is not specifically limited in this embodiment of this application.

An embodiment of this application further provides a computer-readable storage medium. The computer-readable storage medium includes computer instructions, and when the computer instructions are run on the foregoing electronic device, the electronic device is enabled to perform functions or steps performed by the electronic device in the foregoing method embodiments.

An embodiment of this application further provides a computer program product. When the computer program product runs on a computer, the computer is enabled to perform the functions or steps performed by the electronic device in the foregoing method embodiments.

Based on the foregoing description of the implementations, a person skilled in the art may clearly understand that for the purpose of convenient and brief description, division into the foregoing functional modules is merely used as an example for description. In actual application, the foregoing functions can be allocated to different functional modules for implementation based on a requirement, that is, an inner structure of an apparatus is divided into different functional modules to implement all or some of the functions described above.

In the several embodiments provided in this application, it should be understood that the disclosed apparatuses and methods may be implemented in other manners. For example, the described apparatus embodiments are merely examples. For example, division into the modules or units is merely logical function division and may be other division during actual implementation. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may be one or more physical units, may be located in one place, or may be distributed on different places. Some or all of the units may be selected based on an actual requirement to achieve the objectives of the solutions of embodiments.

In addition, functional units in embodiments of this application may be integrated into one processing unit, or each of the units may exist alone physically, or two or more units may be integrated into one unit. The integrated unit may be implemented in a form of hardware, or may be implemented in a form of a software function unit.

When the integrated unit is implemented in the form of a software functional unit and sold or used as an independent product, the integrated unit may be stored in a readable storage medium. Based on such an understanding, the technical solutions of embodiments of this application essentially, or the part contributing to the conventional technology, or all or some of the technical solutions may be implemented in a form of a software product. The software product is stored in a storage medium and includes several instructions for instructing a device (which may be a single-chip microcomputer, a chip, or the like) or a processor (processor) to perform all or some of the steps of the methods described in embodiments of this application. The foregoing storage medium includes any medium, for example, a USB flash drive, a removable hard disk, a read-only memory (read-only memory, ROM), a random access memory (random access memory, RAM), a magnetic disk, an optical disc, or the like that can store program code.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A detection system, configured to detect a physiological parameter, comprising a patch and an electronic device, wherein
the patch comprises a substrate, a first electrode interface, an adhesive layer, and a first microneedle;
the first electrode interface is disposed on a first surface of the substrate;
the adhesive layer is disposed on a second surface of the substrate, and the second surface faces away from the first surface;
the first microneedle is disposed on the second surface of the substrate, wherein a first electrode is disposed on the first microneedle, and the first electrode is coupled to the first electrode interface;
the electronic device comprises an electrochemical sensing circuit and a first external interface; and
the first external interface is disposed on a back surface of the electronic device, and the first external interface is coupled to the electrochemical sensing circuit, wherein when the electronic device is worn with the back surface of the electronic device facing a human body, the first external interface and the first electrode interface are electrically connected, and an electrical signal is transmitted between the patch and the electronic device.

2. The detection system according to claim 1, wherein a second electrode is further disposed on the first microneedle, the patch further comprises a second electrode interface, and the second electrode interface is disposed on the first surface of the substrate, wherein the second electrode is coupled to the second electrode interface; and
the electronic device further comprises a second external interface, the second external interface is disposed on the back surface of the electronic device, and the second external interface is coupled to the electrochemical sensing circuit, wherein when the electronic device is worn with the back surface of the electronic device facing the human body, the second external interface and the second electrode interface are electrically connected, and an electrical signal is transmitted between the patch and the electronic device.

3. The detection system according to claim 1, wherein the patch further comprises a second microneedle and a second electrode interface;
the second microneedle is disposed on the second surface of the substrate;
the second electrode interface is disposed on the first surface of the substrate, wherein a second electrode is disposed on the second microneedle, and the second electrode is coupled to the second electrode interface; and
the electronic device further comprises a second external interface, the second external interface is disposed on the back surface of the electronic device, and the second external interface is coupled to the electrochemical sensing circuit, wherein when the electronic device is worn with the back surface of the electronic device facing the human body, the second external interface and the second electrode interface are electrically connected, and an electrical signal is transmitted between the patch and the electronic device.

4. The detection system according to any one of claims 1 to 3, wherein the first electrode comprises any one of a work electrode and a reference electrode.

5. The detection system according to claim 4, wherein the second electrode comprises any one of the work electrode and the reference electrode, and the second electrode is different from the first electrode.

6. The detection system according to any one of claims 2 to 5, wherein the first electrode interface and the second electrode interface have magnetism, or the first electrode interface and the second electrode interface are magnetic materials; and
the first external interface and the second external interface have magnetism, or the first external interface and the second external interface are magnetic materials.

7. The detection system according to any one of claims 1 to 6, wherein the patch further comprises:
a water retaining strip, wherein the water retaining strip is disposed on the first surface of the substrate, and the water retaining strip surrounds the first electrode interface.

8. The detection system according to any one of claims 1 to 7, wherein the patch further comprises:
an application mark, wherein the application mark is disposed on the first surface of the substrate, and the application mark indicates an application direction of the patch.

9. The detection system according to any one of claims 2 to 8, wherein the first electrode interface and the second electrode interface are concentrically disposed ring-shaped electrode interfaces; and
the first external interface and the second external interface are concentrically disposed ring-shaped external interfaces.

10. The detection system according to any one of claims 1 to 9, wherein a shape of the first electrode comprises a columnar shape, a planar shape, or a wire-wound shape.

11. The detection system according to any one of claims 1 to 10, wherein the first electrode comprises one or more of the following materials: lamp black carbon, glassy carbon, graphite, silver, silver chloride, platinum, palladium, a platinum-iridium alloy, titanium, gold, and iridium.

12. The detection system according to any one of claims 1 to 11, wherein a length of the first microneedle is from 1 to 5 mm, and a diameter of the first microneedle is from 7 to 400 µm.

13. The detection system according to any one of claims 1 to 12, wherein a material of the first microneedle comprises stainless steel, platinum, or a polymer material.

14. The detection system according to any one of claims 1 to 13, wherein
the patch further comprises a first magnetic interface, wherein the first magnetic interface is disposed on the first surface of the substrate, and the first magnetic interface has magnetism, or the first magnetic interface is a magnetic material;
the electronic device further comprises a second magnetic interface disposed on the back surface of the electronic device, wherein the second magnetic interface has magnetism, or the second magnetic interface is a magnetic material; and
when the electronic device is worn with the back surface of the electronic device facing the human body, the first magnetic interface and the second magnetic interface are engaged.

15. A patch, configured to detect a physiological parameter, comprising:
a substrate;
a first electrode interface, wherein the first electrode interface is disposed on a first surface of the substrate, and the first electrode interface is configured to: after being electrically connected to an electronic device, transmit an electrical signal between the patch and the electronic device;
an adhesive layer, wherein the adhesive layer is disposed on a second surface of the substrate, and the second surface faces away from the first surface; and
a first microneedle, wherein the first microneedle is disposed on the second surface of the substrate, wherein
a first electrode is disposed on the first microneedle, and the first electrode is coupled to the first electrode interface.

16. The patch according to claim 15, wherein a second electrode is further disposed on the first microneedle, and the patch further comprises:
a second electrode interface, wherein the second electrode interface is disposed on the first surface of the substrate, wherein
the second electrode is coupled to the second electrode interface.

17. The patch according to claim 15, wherein the patch further comprises:
a second microneedle, wherein the second microneedle is disposed on the second surface of the substrate; and
a second electrode interface, wherein the second electrode interface is disposed on the first surface of the substrate, wherein
a second electrode is disposed on the second microneedle, and the second electrode is coupled to the second electrode interface.

18. The patch according to any one of claims 15 to 17, wherein the first electrode comprises any one of a work electrode and a reference electrode.

19. The patch according to claim 18, wherein the second electrode comprises any one of the work electrode and the reference electrode, and the second electrode is different from the first electrode.

20. The patch according to any one of claims 16 to 19, wherein the first electrode interface and the second electrode interface have magnetism, or the first electrode interface and the second electrode interface are magnetic materials.

21. The patch according to any one of claims 15 to 20, wherein the patch further comprises:
a water retaining strip, wherein the water retaining strip is disposed on the first surface of the substrate, and the water retaining strip surrounds the first electrode interface.

22. The patch according to any one of claims 15 to 21, wherein the patch further comprises:
an application mark, wherein the application mark is disposed on the first surface of the substrate, and the application mark indicates an application direction of the patch.

23. The patch according to any one of claims 15 to 22, wherein the first electrode interface and the second electrode interface are concentrically disposed ring-shaped electrode interfaces.

24. The patch according to any one of claims 15 to 23, wherein a shape of the first electrode comprises a columnar shape, a planar shape, or a wire-wound shape.

25. The patch according to any one of claims 15 to 24, wherein the first electrode comprises one or more of the following materials: lamp black carbon, glassy carbon, graphite, silver, silver chloride, platinum, palladium, a platinum-iridium alloy, titanium, gold, and iridium.

26. The patch according to any one of claims 15 to 25, wherein a length of the first microneedle is from 1 to 5 mm, and a diameter of the first microneedle is from 7 to 400 µm.

27. The patch according to any one of claims 15 to 26, wherein a material of the first microneedle comprises stainless steel, platinum, or a polymer material.

28. The patch according to any one of claims 15 to 27, further comprising a first magnetic interface, wherein the first magnetic interface is disposed on the first surface of the substrate, and the first magnetic interface has magnetism, or the first magnetic interface is a magnetic material.

29. An electronic device, configured to detect a physiological parameter, comprising:
an electrochemical sensing circuit; and
a first external interface, wherein the first external interface is disposed on a back surface of the electronic device, and the first external interface is coupled to the electrochemical sensing circuit, wherein when the electronic device is worn with the back surface of the electronic device facing a human body, the first external interface and a first electrode interface of a patch are electrically connected, and an electrical signal is transmitted between the patch and the electronic device.

30. The electronic device according to claim 29, wherein the first external interface has magnetism, or the first external interface is a magnetic material.

31. The electronic device according to claim 29, wherein the electronic device further comprises a second external interface, the second external interface is disposed on the back surface of the electronic device, and the second external interface is coupled to the electrochemical sensing circuit, wherein when the electronic device is worn with the back surface of the electronic device facing the human body, the second external interface and a second electrode interface of the patch are electrically connected, and an electrical signal is transmitted between the patch and the electronic device.

32. The electronic device according to any one of claims 29 to 31, further comprising:
a second magnetic interface disposed on the back surface of the electronic device, wherein the second magnetic interface has magnetism, or the second magnetic interface is a magnetic material.

33. The electronic device according to claim 31, wherein the first external interface and the second external interface are concentrically disposed ring-shaped external interfaces.
